# EUROPEAN PATENT SPECIFICATION

(11) **EP 4 570 290 B1**
(45) Date of publication and mention of the grant of the patent: **25.11.2026**
(21) Application number: 25173130.3
(22) Date of filing: 31.10.2019
(51) Int. Cl.: A61B 5/00, A61M 16/00, A61M 16/06

(54) **SYSTEM AND METHOD FOR VARYING DATA VOLUME TRANSMITTED TO EXTERNAL SOURCE**
SYSTEM UND VERFAHREN ZUR VERÄNDERUNG DER ZU EINER EXTERNEN QUELLE GESENDETEN DATENMENGE
SYSTÈME ET PROCÉDÉ POUR FAIRE VARIER LE VOLUME DE DONNÉES TRANSMISES À UNE SOURCE EXTERNE

(30) Priority: 31.10.2018 US 201862753828 P; 16.09.2019 US 201962900982 P
(43) Date of publication of application: 18.06.2025
(62) Document number(s) of the earlier application(s) in accordance with Art. 76 EPC: 19877680.9 / 3 873 329
(73) Proprietor: ResMed Inc., San Diego CA 92123 (US)
(72) Inventor: KENNEDY, Colin Bradley, San Diego, California, 92123 (US); NATHWANI, Rehana, San Diego, California, 92123 (US); WREN, Michael, Dublin, D18 T6T7 (IE); SHOULDICE, Redmond, Dublin, D18 T6T7 (IE)
(74) Representative: Mathys & Squire

(56) References cited:
- US-A1- 2009 076 405
- US-A1- 2013 253 355
- US-A1- 2014 194 762
- US-A1- 2017 296 057
- US-A1- 2017 311 879
- US-A1- 2017 332 931

## Description

### 1 BACKGROUND OF THE TECHNOLOGY

### 1.1 PRIORITY CLAIM

This application claims priority to U.S. Provisional Application No. 62/753,828, titled "System For Varying Data Volume Transmitted to External Source" and filed on October 31, 2018, and U.S. Provisional Application No. 62/900,982, titled "SYSTEM AND METHOD FOR CLOUD BASED HEALTH CARE ANALYSIS FROM OPERATIONAL DATA COLLECTED FROM A RESPIRATORY THERAPY DEVICE" filed September 16, 2019.

### 1.2 FIELD OF THE TECHNOLOGY

The present technology relates to one or more of the screening, diagnosis, monitoring treatment, prevention and amelioration of respiratory-related disorders. The present technology also relates to medical devices or apparatus, and varying data transmitted from medical devices to outside storage systems.

### 1.3 BACKGROUND DESCRIPTION

The respiratory system of the body facilitates gas exchange. The nose and mouth form the entrance to the airways of a patient. The airways include a series of branching tubes, which become narrower, shorter and more numerous as they penetrate deeper into the lung. The prime function of the lungis gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas. Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Chronic diseases are the leading causes of death and disability worldwide. By 2020 their contribution is expected to rise to 73% of all deaths and 60% of the global burden of disease. This is associated with soaring costs of health care. Chronic diseases are the primary driver of health care costs, accounting for ninety cents (90¢) of every dollar spent in the U.S. This cost is related to an aging population. In 2015, one out of eight people worldwide was aged 60 years or over. By 2030, it is estimated that one in six people will be aged 60 years or over.

Chronic disease management (CDM) applications and services exist to track chronic conditions. One example is the Twine service which was recently acquired by FitBit. Further, telehealth services exist to monitor people at home using wired and wireless sensors and cellular data connections. There is a trend toward technology solutions and increasing acceptance for older people with a higher prevalence of sleep-disordered breathing and co-morbid conditions. Such solutions may include smartphone applications, continuous positive airway pressure (CPAP) usage applications, wearable activity monitors, Internet of Medical things (IoMT), artificial intelligence (AI), and communication technologies such as Wi-Fi, Bluetooth, 4G, and 5G.

Current chronic disease management applications tend to have poor compliance as the user has to regularly interact with them and may provide inaccurate information to the application. This is especially the case if the user believes certain answers may change the price/cost or availability of insurance or care. In other cases, a user simply cannot judge if their condition is worsening or not, or which symptoms are more important than others. In other words, the systems remove boundaries between the user and their health management.

A range of therapies have been used to treat or ameliorate such conditions. Furthermore, otherwise healthy individuals may take advantage of such therapies to prevent respiratory disorders from arising. However, these have a number of shortcomings.

Various therapies, such as Continuous Positive Airway Pressure (CPAP) therapy, Non-invasive ventilation (NIV) and Invasive ventilation (IV) have been used to treat one or more of the above respiratory disorders.

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may electnotto comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

These respiratory therapies may be provided by a therapy system or device. Such systems and devices may also be used to screen, diagnose, or monitor a condition without treating it. A respiratory pressure therapy (RPT) device may be used individually or as part of a system to deliver one or more of a number of therapies described above, such as by operating the device to generate a flow of air for delivery to an interface to the airways. A respiratory therapy system may comprise a Respiratory Pressure Therapy Device (RPT device), an air circuit, a humidifier, a patient interface, an oxygen source, and data management.

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

Due to the difficulties of user compliance, such CPAP devices are configured to transmit certain operational data during operation by the user. Such data allows determination of whether the patient prescribed with respiratory therapy has been "compliant", e.g., that the patient has used their respiratory pressure therapy device according to one or more "compliance rules." One example of a compliance rule for CPAP therapy is that a patient, in order to be deemed compliant, is required to use the respiratory pressure therapy device for at least four hours a night for at least twenty-one (21) of thirty (30) consecutive days. In order to determine a patient's compliance, a provider of the respiratory pressure therapy device, such as a health care provider, may manually obtain data describing the patient's therapy using the respiratory pressure therapy device. The provider may calculate the usage over a predetermined time period, and compare it with the compliance rule. Once the health care provider has determined that the patient has used their respiratory pressure therapy device according to the compliance rule, the health care provider may notify a third party, such as a payor, that the patient is compliant.

However, currently, such devices transmit very limited summary breathing data, for example, only ten (10) kilobytes per night or three hundred (300 kilobytes) a month, to remote locations via networks such as the cloud. This data is typically supplied using one way communications only, or with very limited bidirectional traffic. Detailed data is generally not stored by current devices, or only stored on a memory card, which may be read manually on a periodic basis. No real time cardiac/chronic disease insights are made available from the limited data as such data is generally only useful to determine the operation of the CPAP device.

It would be advantageous for a system to use existing operational data collected by a respiratory pressure therapy device for disease management. There is a need for a system that increases the rate of data collection from a respiratory pressure therapy device for analysis of a health condition based on a triggering event. There is another need for integration of additional external data with data collected from respiratory pressure therapy devices to determine health conditions of a patient.

US 2017/332931 A1 discloses systems and methods that limit transmission of forms of patient data that require relatively high bandwidth (e.g., high resolution data) by instead transmitting forms of patient data that require relatively low bandwidth (e.g., low resolution data). In some of these examples, the systems and methods transmit low resolution data, receive guidance that indicates particular portions of high resolution data to transit, and transmit only those portions. In these examples, the guidance may be received from user input that is informed by the low resolution data.

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability. Specifically, this disclosure relates to modulating data used in the screening, diagnosis, monitoring, amelioration, treatment or prevention of a respiratory disorder.

This disclosure is directed toward a cloud-connected chronic disease management platform. The system includes a respiratory pressure therapy device such as a cloud-connected positive airway pressure (PAP) device. The PAP device includes mechanical components that traditionally are used to treat sleep-disordered breathing (SDB). For example, when using PAP device therapy, the patient and/or payor (such as an integrated-care provider) not only receives data from the device to get insights into the efficacy of sleep-disordered breathing treatment and compliance, but also insights into patient health conditions such as atrial fibrillation, potential stroke risk, hypertension (including drug resistant hypertension), heart failure, type 2 diabetes and obesity (body fat content) information. Cardiac information can be derived from heart rate, beat information, cardiac output, and so forth. This is preferentially derived from the mask interface to the patient, and the machine and related sensors. The system can connect to other data such as that contained in an electronic health record (EHR) of the patient as part of an overall chronic disease management offering.

A respiratory therapy system may include a respiratory pressuretherapy (RPT) device, an air circuit, a humidifier, a patient interface, an oxygen source, and data management. The RPT device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as high flow therapy). Thus respiratory pressure therapy devices may also act as flow therapy devices. Examples of respiratory pressure therapy devices include PAP devices and ventilators.

Current respiratory pressure therapy devices such as a CPAP device collect data from patients during use. Such collected data is provided to a central hub such as the device itself or a mobile computing device for transmission to a cloud-based analysis engine for further analysis relating primarily to patient health. The disclosed platform uses the increased collection of the CPAP data to provide health condition analysis for the patient. The determined health condition of the patient may be used for additional treatment, evaluating treatment, patient or care giver alerts, and other purposes.

According to an example of the disclosure, a patient treatment device is provided, including a memory, a first sensor configured to collect a first type of data from a patient being stored in the memory, a controller coupled to the first sensor, and a transmitter is disclosed. The controller is operable to control the memory to store low resolution data of the first type of data collected at a lower sampling rate, determine the occurrence of a triggering event, and control the memory to store higher resolution data of the first type of data collected at a higher sampling rate. The transmitter is controlled by the controllerto send the low resolution data or the high resolution data to an external system.

Accordingtc an example of the disclosure, a method to modulate the transmission of patient data from a patient treatment device to an external system is provided. The patient treatment device includes a first sensor coupled to a patient, a memory, and a transmitter. The method comprises collecting a first type from the first sensor, storing low resolution data collected from the first sensor at a lower sampling rate, determining the occurrence of a triggering event, storing high resolution data collected from the first sensor at a higher sampling rate; and transmitting either the low resolution data or the high resolution data to an external device via the transmitter.

In one example, a method for transmitting varying data volume of patient data to an external system is provided, the patient data being stored in a memory, and the patient data including low resolution data and high resolution data, the method including: sending low resolution data from the memory to the external system by a transmitter internal or external of a respiratory pressure therapy device; determining the occurrence of an event based on the low resolution data; and on the event occurring, changing the transmitter to send high resolution data to the external system by a controller internal or external of the respiratory pressure therapy device.

One disclosed example is a system for monitoring the health of a patient. The system includes a respiratory therapy device having a transmitter and an air control device to provide respiratory therapy to a patient. The respiratory therapy device collects operational data and transmits the collected operational data. A health analysis engine is in communication with the respiratory therapy device. The health analysis engine receives the collected data to determine a health condition of the patient based on the collected data.

Another disclosed example is a method for monitoring health of a patient using a respiratory therapy device. Operational data of the respiratory therapy device is collected. The collected data is transmitted to a health analysis engine. A health condition of the patient is determined by the health analysis engine based on the collected data.

The above summary is not intended to represent each embodiment or every aspect of the present disclosure. Rather, the foregoing summary merely provides an example of some of the aspects and features set forth herein. The above features and advantages, and other features and advantages of the present disclosure will be readily apparent from the following detailed description of representative embodiments and modes for carrying out the present disclosure.

### SUMMARY OF THE INVENTION

The present invention provides a patient treatment device as defined in claim 1 and a method to modulate the transmission of patient data from a patient treatment device to an external system as defined in claim 14. Preferred embodiments of the invention are defined in the dependent claims.

### 3 BRIEF DESCRIPTION OF THE DRAWINGS

The present technology is illustrated by way of example, and not by way of limitation, in the figures of the accompanying drawings, in which like reference numerals refer to similar elements including:

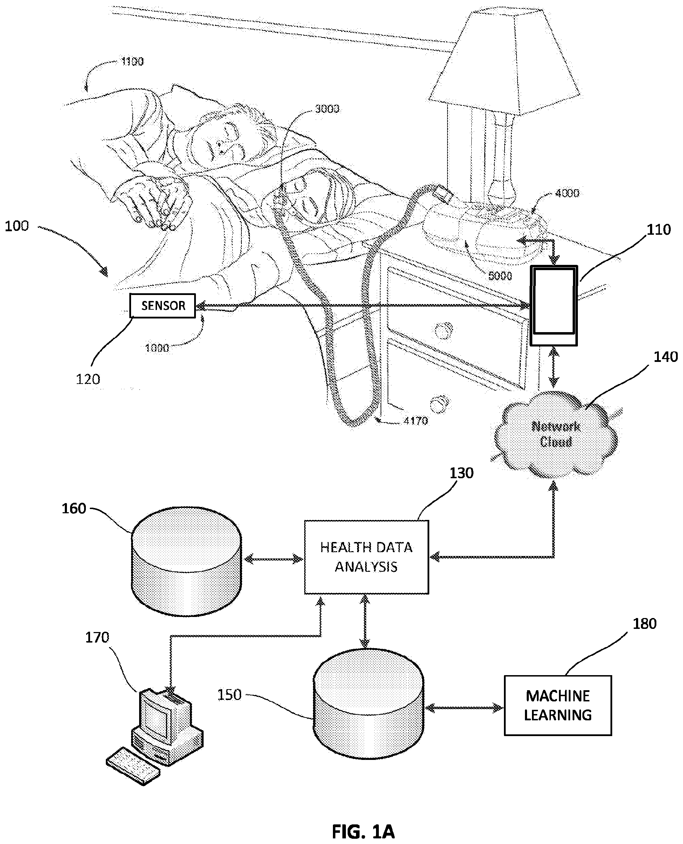

FIG. 1A shows a system including a patient 1000 wearing a patient interface 3000, in the form of nasal pillows, receiving a supply of air at positive pressure from a respiratory pressure therapy (RPT) device 4000. In this example, the respiratory pressure therapy device may include a PAP device, a non-invasive ventilation (NIV) device, or an adaptive support ventilation (ASV) device. Of course, other devices may also perform the functions of the RPT device 4000 described herein. Air from the RPT device 4000 is humidified in a humidifier 5000, and passes along an air circuit 4170 to the patient 1000. A bed partner 1100 is also shown. The patient 1000 is sleeping in a supine sleeping position. A health analysis system collects data from the RPT device 4000 used by the patient 1000
FIG. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
FIG. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
FIG. 2 shows an overview of a human respiratory system including the nasal and oral cavities, the larynx, vocal folds, oesophagus, trachea, bronchus, lung, alveolar sacs, heart and diaphragm.

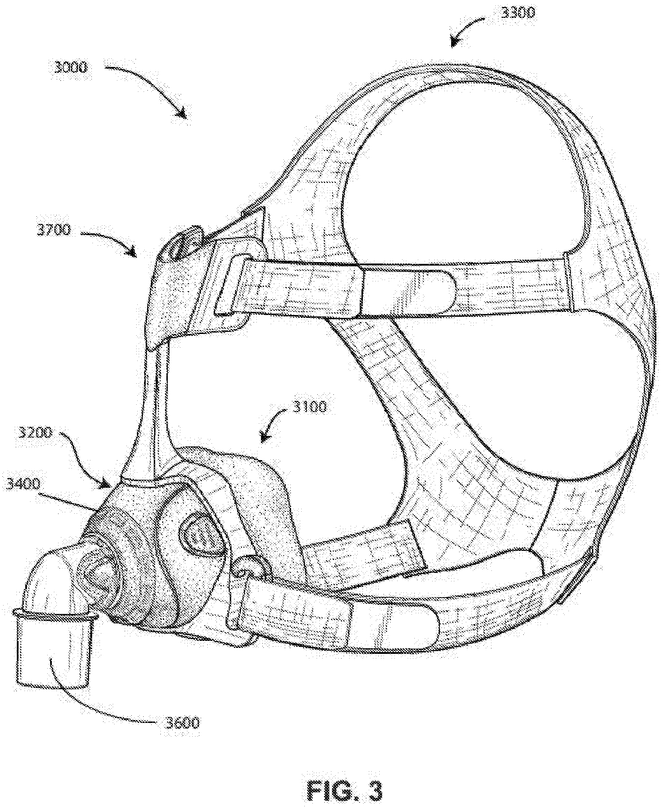

FIG. 3 shows a patient interface in the form of a nasal mask in accordance with one form of the present technology.
FIG. 4A shows an RPT device in accordance with one form of the present technology.
FIG. 4B is a schematic diagram of the pneumatic path of an RPT device in accordance with one form of the presenttechnology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.
FIG. 4C is a schematic diagram of the electrical components of an RPT device in accordance with one form of the present technology.
FIG. 4D is a schematic diagram of the algorithms implemented in an RPT device in accordance with one form of the present technology.
FIG. 4E is a flow chart illustrating a method carried out by the therapy engine module of Fig. 4D in accordance with one form of the present technology.
FIG. 5A shows an isometric view of a humidifier in accordance with one form of the present technology.
FIG. 5B shows an isometric view of a humidifier in accordance with one form of the present technology, showing a humidifier reservoir 5110 removed from the humidifier reservoir dock 5130.
FIG. 6 shows a model typical breath waveform of a person while sleeping.
FIG. 7A shows a patient undergoing polysomnography (PSG). The patient is sleeping in a supine sleeping position.
FIG. 7B shows a monitoring apparatus for monitoring the condition of a patient. The patient is sleeping in a supine sleeping position.
FIG. 7C is a block diagram illustrating a screening / diagnosis / monitoring device that may be used to implement PSG.
FIG. 8A is a block diagram of a system that receives modulated data from a monitoring apparatus.
FIG. 8B is a block diagram of an alternate system that receives modulated data from a monitoring apparatus.
FIG. 9 is a flow diagram of the process of modulating data transmission from the monitoring apparatus described in FIG. 4A and 7A.
FIG. 10 is a flow diagram of the routine to increase collection of data based on a triggering event and provide health analysis from the monitoring apparatus described in FIG. 4A and 7A.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

The present disclosure relates to a system that leverages operational data collected by respiratory pressure therapy devices to allow for analysis of patient health conditions in a cloud-based engine. The system provides a connected-care service value in the health care (and particularly Integrated Care) market, whereby reducing the burden of care, particularly for those with sleep-disordered breathing and co-morbidities, by incorporating sensing technology in positive-airway-pressure-enabled devices and services, supported by communications technologies such as Wi-Fi, Bluetooth, 3G, 4G/LTE, 5G/New Radio, fixed wireless, satellite, and beyond.

An example respiratory pressure therapy device may monitor and collect operational data such as the motor voltage, RPM, air flow, and mask leak. The example respiratory pressure therapy device may also monitor and collect physiological data via cardiac signals derived from a microphone in or near the tube, from the mask signal, from an optical sensor near or on the face (such as in the mask), from electrodes (in or on the mask, headgear), from a connected patch with electrical or optical sensing, from a smart watch, bracelet, or ring. Gas analysis could be at the mask or sampled in the tubing or device. Sweat analysis could be at the mask. Data could also be integrated from other devices, such as a smart inhaler, used by the patient.

Effectively, the example disclosed respiratory data collection platform could encompass chronic disease management in addition to sleep-disordered breathing therapy, such as Hypertension, Stroke, COPD, Congestive heart failure (CHF), arrhythmias such as Atrial fibrillation, Asthma, Diabetes, as well as dementia, mental health issues (e.g., depression, bipolar disorder, ADHD) and other disorders.

### 4.1 THERAPY

In one form, the present technology comprises a method for treating a respiratory disorder comprising the step of applying positive pressure to the entrance ofthe airways of a patient 1000 in FIGs. 1A-1C. In certain examples of the present technology, a supply of air at positive pressure is provided to the nasal passages of the patient via one or both nares. In certain examples of the present technology, mouth breathing is limited, restricted or prevented.

### 4.2 TREATMENT SYSTEMS

In one form, the present technology comprises an apparatus or device for treating a respiratory disorder. The apparatus or device may comprise an RPT device 4000 for supplying pressurised air to the patient 1000 via an air circuit 4170 to a patient interface 3000 or 3800.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

An unsealed patient interface 3800, in the form of a nasal cannula, includes nasal prongs 3810a, 3810b which can deliver air to respective nares of the patient 1000. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3 800 lead to an RT device that generates the flow of air at high flow rates. The "vent" at the unsealed patient interface 3800, through which excess airflow escapes to ambient, is the passage between the end of the prongs 3810a and 3810b of the cannula 3800 via the patient's nares to atmosphere.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of airin a range of -20 L/min to +150 L/min while maintaining a positive pressure of at least 6 cmH₂O, or at least 10cmH₂O, or at least 20 cmH₂O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

An RPT device may comprise one or more of the following components in an integral unit. In an alternative form, one or more of the following components may be located as respective separate units.

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

An RPT device in accordance with one form of the present technology may include a muffler 4120, or a plurality of mufflers 4120.

In one form of the presenttechnology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH₂O to about 20 cmH₂O, or in other forms up to about 30 cmH₂O. The blower may be as described in any one of the following patents or patent applications: U.S. PatentNo. 7,866,944; U.S. Patent No. 8,638,014; U.S. Patent No. 8,636,479; and PCT Patent Application Publication No. WO 2013/020167.

The pressure generator 4140 is under the control of the therapy device controller 4240. In other forms, a pressure generator 4140 may be a piston-driven pump, a pressure regulator connected to a high pressure source (e.g. compressed air reservoir), or a bellows.

Transducers may be internal of the RPT device 4000, or external of the RPT device. External transducers may be located for example on or form part of the air circuit, e.g., the patient interface. External transducers may be in the form of non-contact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800. In one form, a signal from a transducer 4270 may be filtered, such as by low-pass, high-pass or band-pass filtering.

A flow rate sensor 4274 in accordance with the present technology may be based on a differential pressure transducer, for example, an SDP600 Series differential pressure transducer from SENSIRION. In one form, a signal representing a flow rate from the flow rate sensor 4274 is received by the central controller 4230.

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC. In one form, a signal from the pressure sensor 4272 is received by the central controller 4230.

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

A power supply 4210 may be located internal or external of the external housing 4010 of the RPT device 4000. In one form of the present technology, power supply 4210 provides electrical power to the RPT device 4000 only. In another form of the present technology, power supply 4210 provides electrical power to both RPT device 4000 and humidifier 5000.

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

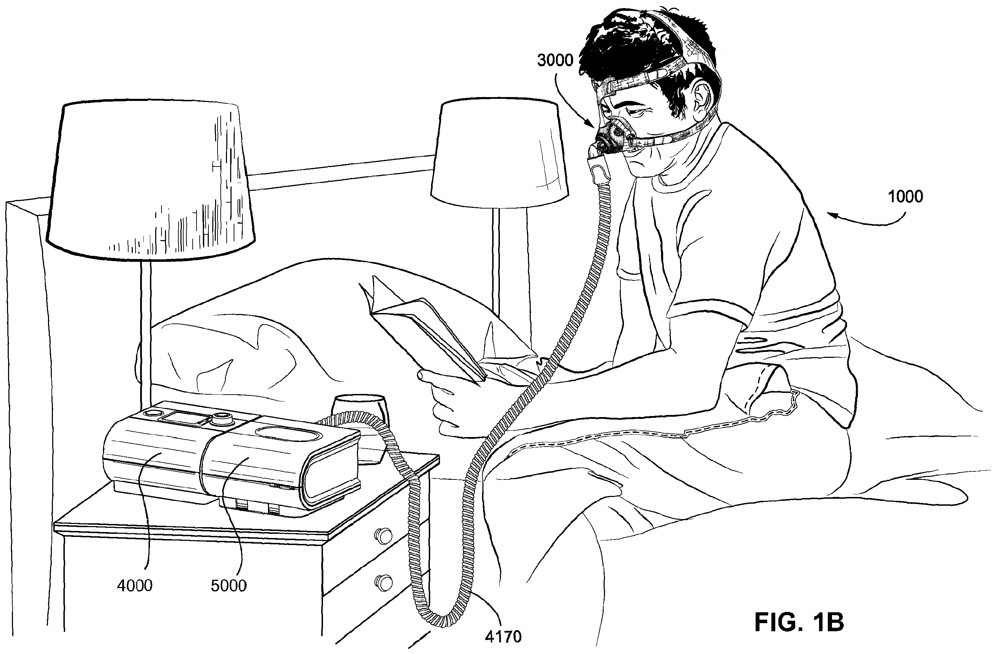

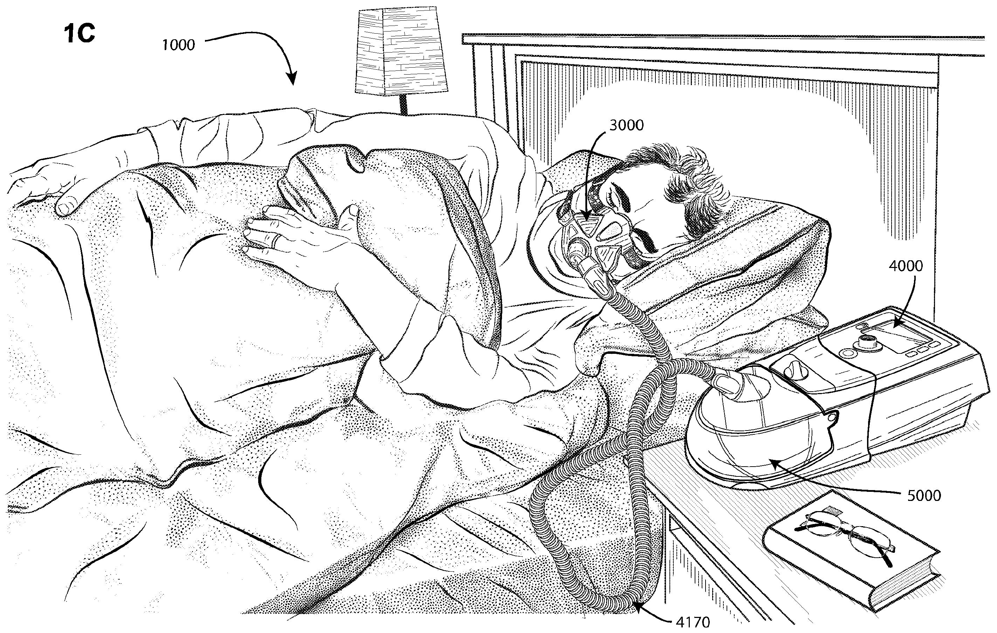

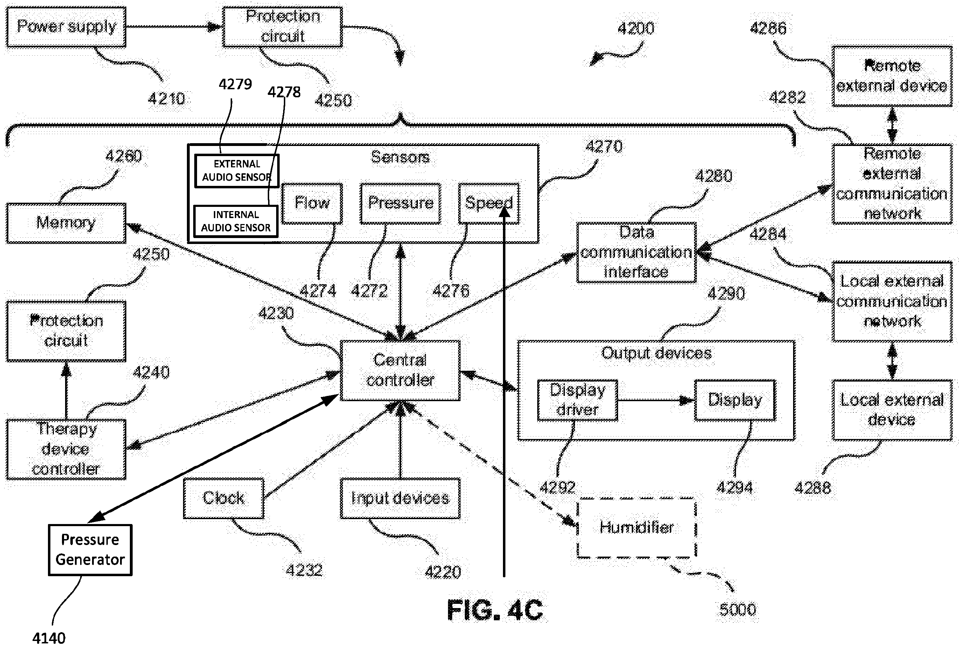

Internal sensors such as the flow rate sensor 4274, a pressure sensor 4272, and a motor speed transducer 4276 may be coupled to the central controller 4230 in FIG. 4C. An optional internal audio sensor 4278 may be embedded in the interface 3000 in FIG. 1 to detect specific patient air sounds. An optional external audio sensor 4279 such as a microphone may be located on the exterior of the RPT device 4000, the interface 3000, or the humidifier 5000 to collect additional audio data. Additional sensors such as a heart rate sensor, an ECG sensor (providing cardiac fiducial parameters, of which peaks could be processed to estimate heart rate, detect arrhythmias and so forth), a pulse oximeter (SpO2) sensor (providing heart rate, oxygen saturation, and potential an estimate of blood pressure from pulse transit time), a blood pressure sensor, a room-temperature sensor, a contact or non-contact body temperature sensor, a room humidity sensor, a proximity sensor, a gesture sensor, a touch sensor, a gas sensor, an air quality sensor, a particulate sensor, an accelerometer, a gyroscope, a tilt sensor, other acoustic sensors such as passive or active SONAR, an ultrasonic sensor, a radio frequency sensor, an accelerometer, a light intensity sensor, a LIDAR sensor, an infrared sensor (passive, transmissive, or reflective), carbon dioxide sensor, a carbon monoxide sensor, or a chemical sensor, may be connected to the central controller 4230 via an external port. Data from such additional sensors may also be collected by the central controller 4230. Data from the sensors 4272, 4274, 4276, 4278, and 4279 may be collected by central controller 4230 on a periodic basis. Such data generally relates to the operational state of the RPT device 4000.

In one form of the present technology, the central controller 4230 is one or a plurality of processors suitable to control an RPT device 4000. Suitable processors may include an x86 INTEL processor, a processor based on ARM^{®} Cortex^{®}-M processor from ARM Holdings such as an STM32 series microcontroller from STMICROELECTRONICS. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit. In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000. The central controller 4230 may be configured to provide output signal(s) to one or more of an output device 4290, a therapy device controller 4240, a data communication interface 4280, and the humidifier 5000.

The controller4230 may collect the data at different rates. For example, duringnormal use, the data may be collected at a low-resolution rate. As will be explained, a triggering event may cause the controller 4230 to start collecting the data at a different collection rate, such as at a higher resolution rate for collecting more data and/or additional types of data in a comparative time period than the low-resolution rate for more detailed analysis of the patient 1000. In this example, the central controller 4230 encodes such data from the sensors in a proprietary data format. The data may also be coded in a standardized data format.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein. As explained above, all data and operations for external sources or the central controller 4230 are generally proprietary to the manufacturer of the RPT device 4000. Thus, the data from the sensors and any other additional operational data is not generally accessible by any other device.

The RPT device 4000 may include a clock 4232 that is connected to the central controller 4230.

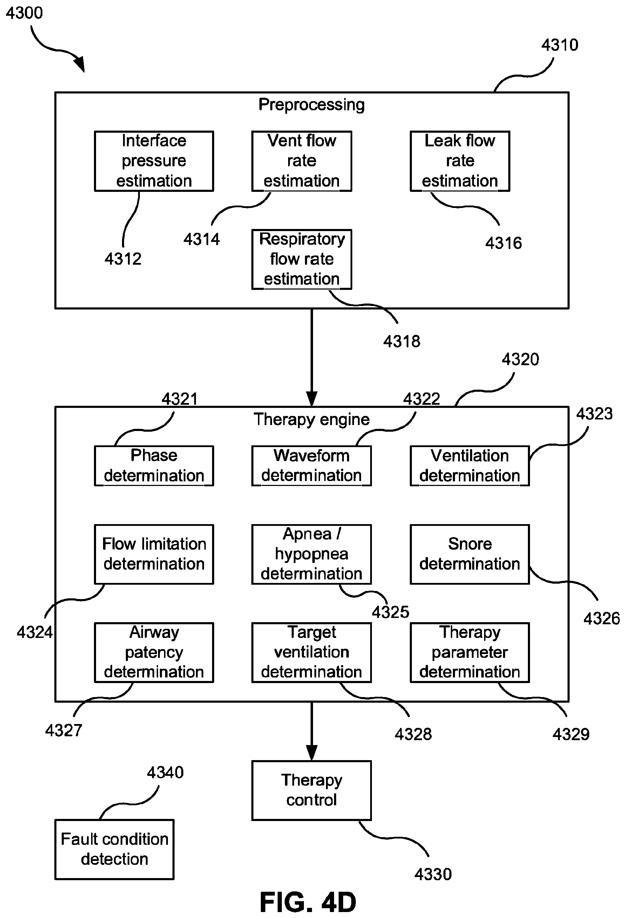

In one form of the present technology, therapy device controller 4240 is a therapy control module 4330 that forms part of the algorithms 4300 executed by the central controller 4230 as shown in FIG. 4D. In one form of the present technology, therapy device controller 4240 is a dedicated motor control integrated circuit. For example, in one form a MC33035 brushless DC motor controller, manufactured by ONSEMI is used.

The one or more protection circuits 4250 in accordance with the present technology may comprise an electrical protection circuit, a temperature and/or pressure safety circuit.

In accordance with one form of the present technology the RPT device 4000 includes memory 4260, e.g., non-volatile memory. In some forms, memory 4260 may include battery powered static RAM. In some forms, memory 4260 may include volatile RAM. The memory 4260 may be located on the PCBA 4202. The memory 4260 may be in the form of EEPROM, or NAND flash. Additionally or alternatively, RPT device 4000 includes a removable form of memory 4260, for example a memory card made in accordance with the Secure Digital (SD) standard. In one form of the present technology, the memory 4260 acts as a non-transitory computer readable storage medium on which is stored computer program instructions expressing the one or more methodologies described herein, such as the one or more algorithms 4300.

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor. In one form, remote external communication network 4282 is the Internet. The data communication interface 4280 may use wired communication (e.g. via Ethernet, or optical fibre) or a wireless protocol (e.g. CDMA, GSM, LTE) to connect to the Internet. In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician. The local external device 4288 may be a personal computer, mobile phone, tablet or remote control.

An output device 4290 in accordance with the present technology may take the form of one or more of a visual, audio and haptic unit. A visual display may be a Liquid Crystal Display (LCD) or Light Emitting Diode (LED) display.

A display driver 4292 receives as an input the characters, symbols, or images intended for display on the display 4294, and converts them to commands that cause the display 4294 to display those characters, symbols, or images.

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

As mentioned above, in some forms of the present technology, the central controller 4230 may be configured to implement one or more algorithms 4300 in FIG. 4D expressed as computer programs stored in a non-transitory computer readable storage medium, such as memory 4260. The algorithms 4300 are generally grouped into groups referred to as modules.

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure *Pm,* the respiratory flow rate *Qr,* and the leak flow rate *Ql.* In various forms of the present technology, the pre-processing module 43 10 comprises one or more of the following algorithms: interface pressure estimation 43 12, vent flow rate estimation 4314, leak flow rate estimation 4316, and respiratory flow rate estimation 4318.

The interface pressure estimation algorithm, 4312 provides as an output an estimated pressure, *Pm,* in the patient interface 3000 or 3800. The vent flow rate estimation algorithm 4314 estimates a vent flow rate of air, *Qv,* from a vent 3400 in a patient interface 3000 or 3800. The leak flow rate estimation algorithm 4316 estimates the leak flow rate *Qlby* calculating an average of the difference between total flow rate *Qt* and vent flow rate *Qv* over a period sufficiently long to include several breathing cycles, e.g. about 10 seconds. The respiratory flow rate estimation algorithm 43 18 estimates a respiratory flow rate of *air, Qr,* to the patient, by subtracting the vent flow rate *Qv* and the leak flow rate *Ql* from the total flow rate *Qt.*

In one form of the presenttechnology, a therapy engine module 4320 receives as inputs one or more of a pressure, *Pm,* in a patient interface 3000 or 3800, and a respiratory flow rate of air to a patient, *Qr,* and provides as an output one or more therapy parameters such as one or more of a treatment pressure *Pt,* an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

The phase determination algorithm 4321 provides as an output a phase Φ of a current breathing cycle of a patient 1000.

The waveform determination algorithm 4322 provides a waveform template Π(Φ) with values in the range [0, 1] on the domain of phase values Φ provided by the phase determination algorithm 4321 to be used by the therapy parameter determination algorithm 4329. The ventilation determination algorithm 4323 receives an input a respiratory flow rate *Qr,* and determines a measure indicative of current patient ventilation, *Vent.* The inspiratory flow limitation determination algorithm 4324 receives as an input a respiratory flow rate signal *Qr* and provides as an output a metric of the extent to which the inspiratory portion of the breath exhibits inspiratory flow limitation.

The apnea/hypopnea determination algorithm 4325 receives as an input a respiratory flow rate signal *Qr* and provides as an output a flag that indicates that an apnea or a hypopnea has been detected. The snore determination algorithm 4326 receives as an input a respiratory flow rate signal *Qr* and provides as an output a metric of the extent to which snoring is present.

The airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

The central controller 4230 takes as input the measure of current ventilation, *Vent,* and executes one or more target ventilation determination algorithms 4328 for the determination of a target value *Vtgt* for the measure of ventilation.

The central controller 4230 executes one or more therapy parameter determination algorithms 4329 for the determination of one or more therapy parameters such as instantaneous treatment pressure *Pt* using the values returned by one or more of the other algorithms in the therapy engine module 4320.

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the presenttechnology, the central controller 4230 executes one or more methods 4340 for the detection of fault conditions. The fault conditions detected by the one or more methods 4340 may include at least one of the following:
- Power failure (no power, or insufficient power)
- Transducer fault detection
- Failure to detect the presence of a component
- Operating parameters outside recommended ranges (e.g. pressure, flow rate, temperature, PaO₂)
- Failure of a test alarm to generate a detectable alarm signal.

Upon detection of the fault condition, the corresponding algorithm 4340 signals the presence of the fault by one or more of the following:
- Initiation of an audible, visual &/or kinetic (e.g. vibrating) alarm
- Sending a message to an external device
- Logging of the incident

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuitis described in United States Patent 8,733,349, whichis incorporated herewithin in its entirety by reference.

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

### 4.6 HUMIDIFIER

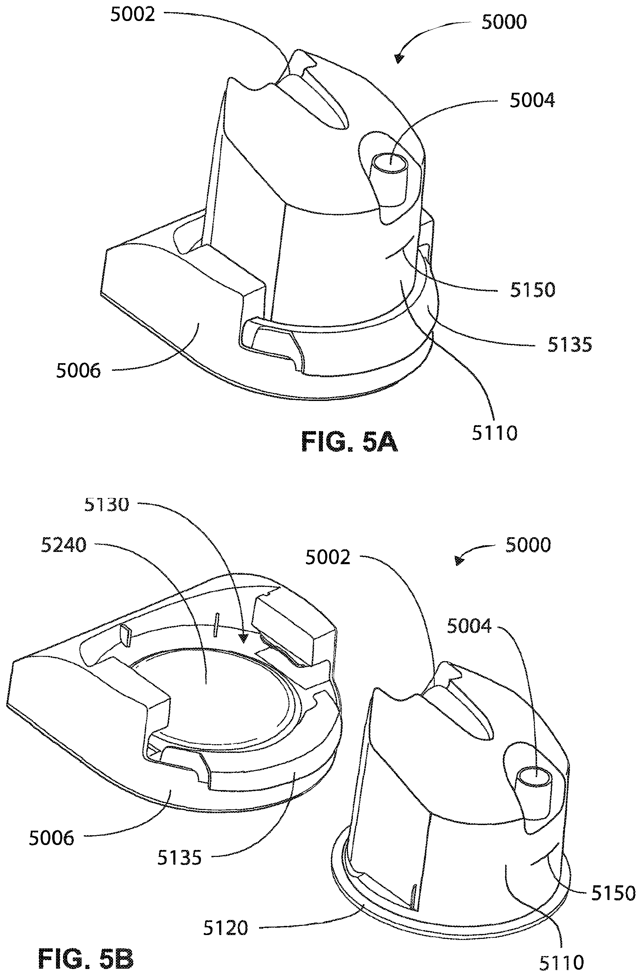

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.7 BREATHING WAVEFORMS

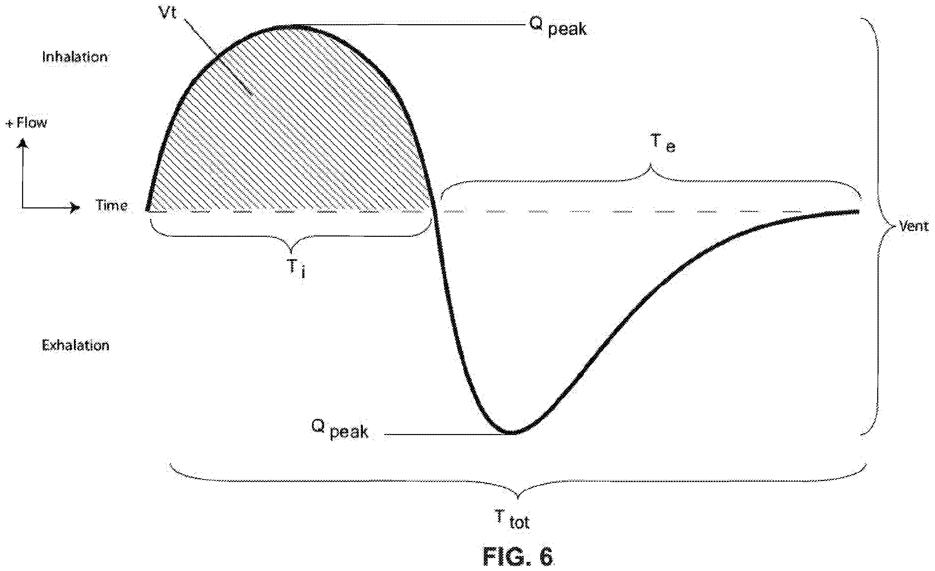

Fig. 6 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 4.8 SCREENING, DIAGNOSIS, MONITORING SYSTEMS

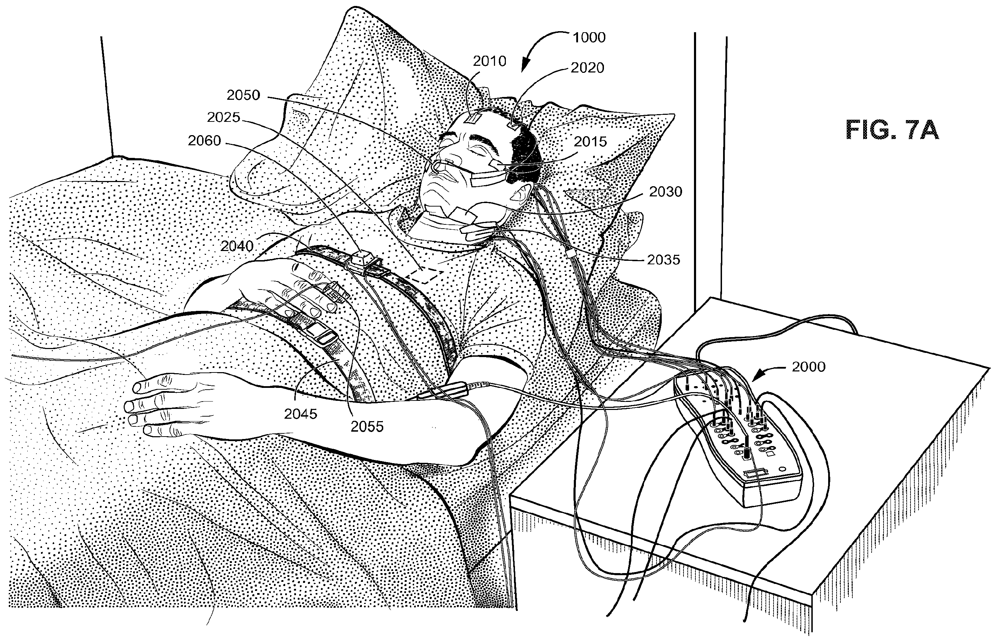

Fig. 7A shows a patient 1000 undergoing polysomnography (PSG). A PSG system comprises a headbox 2000 which receives and records signals from the following sensors: an EOG electrode 2015; an EEG electrode 2020; an ECG electrode 2025; a submental EMG electrode 2030; a snore sensor 2035; a respiratory inductance plethysmogram (respiratory effort sensor) 2040 on a chest band; a respiratory inductance plethysmogram (respiratory effort sensor) 2045 on an abdominal band; an oro-nasal cannula 2050 with oral thermistor; a photoplethy smograph (pulse oximeter) 2055; and a body position sensor 2060. The electrical signals are referred to a ground electrode (ISOG) 2010 positioned in the centre of the forehead.

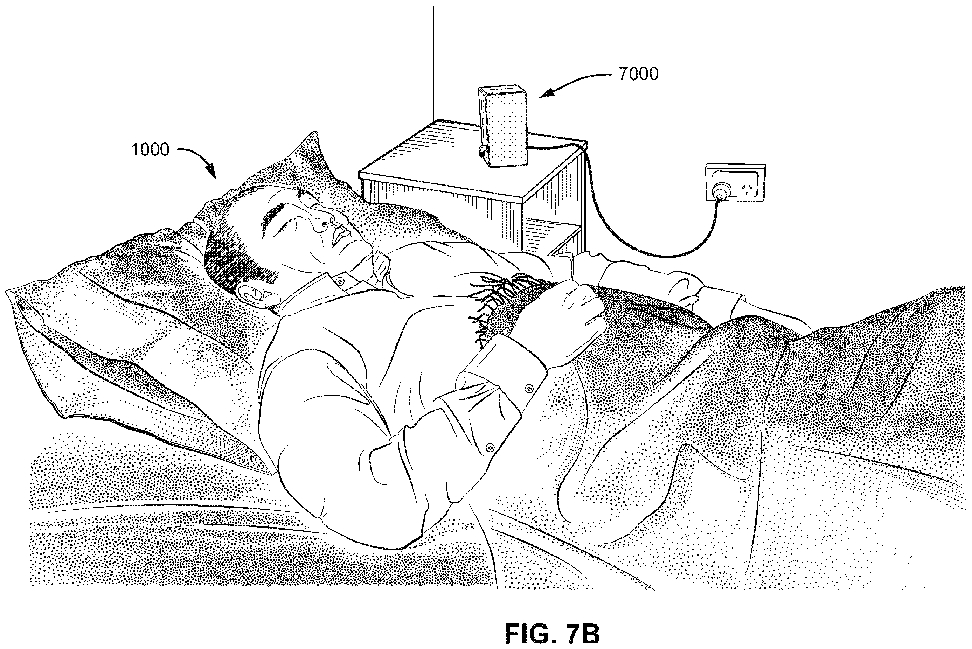

One example of a monitoring apparatus 7100 for monitoring the respiration of a sleeping patient 1000 is illustrated in Fig. 7B. The monitoring apparatus 7100 contains a contactless motion sensor generally directed toward the patient 1000. The motion sensor is configured to generate one or more signals representing bodily movement of the patient 1000, from which may be obtained a signal representing respiratory movement of the patient.

Respiratory polygraphy (RPG) is a term for a simplified form of PSG without the electrical signals (EOG, EEG, EMG), snore, or body position sensors. RPG comprises at least a thoracic movement signal from a respiratory inductance plethysmogram (movement sensor) on a chest band, e.g. the movement sensor 2040, a nasal pressure signal sensed via a nasal cannula, and an oxygen saturation signal from a pulse oximeter, e.g. the pulse oximeter 2055. The three RPG signals, or channels, are received by an RPG headbox, similar to the PSG headbox 2000.

In certain configurations, a nasal pressure signal is a satisfactory proxy for a nasal flow rate signal generated by a flow rate transducer in-line with a sealed nasal mask, in that the nasal pressure signal is comparable in shape to the nasal flow rate signal. The nasal flow rate in turn is equal to the respiratory flow rate if the patient's mouth is kept closed, i.e. in the absence of mouth leaks.

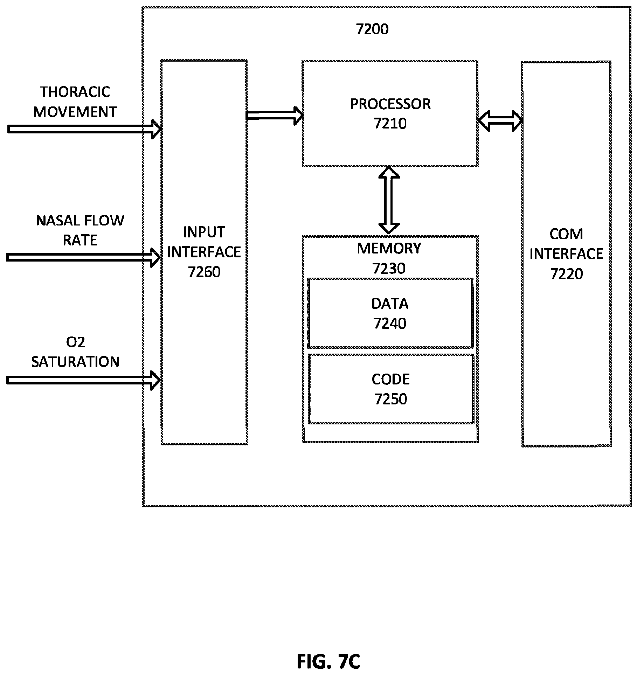

Fig. 7C is a block diagram illustrating a screening/ diagnosis / monitoring device 7200 that may be used to implement an RPG headbox in an RPG screening / diagnosis / monitoring system. The screening / diagnosis / monitoring device 7200 receives the three RPG channels mentioned above (a signal indicative of thoracic movement, a signal indicative of nasal flow rate, and a signal indicative of oxygen saturation) at a data input interface 7260. The screening / diagnosis / monitoring device 7200 also contains a processor 7210 configured to carry out encoded instructions. The screening/ diagnosis / monitoring device 7200 also contains a non-transitory computer readable memory / storage medium 7230.

Memory 7230 may be the screening / diagnosis / monitoring device 7200's internal memory, such as RAM, flash memory or ROM. In some implementations, memory 7230 may also be a removable or external memory linked to screening / diagnosis / monitoring device 7200, such as an SD card, server, USB flash drive or optical disc, for example. In other implementations, memory 7230 can be a combination of external and internal memory. Memory 7230 includes stored data 7240 and processor control instructions (code) 7250 adapted to configure the processor 7210 to perform certain tasks. Stored data 7240 can include RPG channel data received by data input interface 7260, and other data that is provided as a component part of an application. Processor control instructions 7250 can also be provided as a component part of an application program. The processor 7210 is configured to read the code 7250 from the memory 7230 and execute the encoded instructions. In particular, the code 7250 may contain instructions adapted to configure the processor 7210 to carry out methods of processing the RPG channel data provided by the interface 7260. One such method may be to store the RPG channel data as data 7240 in the memory 7230. Another such method may be to analyse the stored RPG data to extract features. The processor 7210 may store the results of such analysis as data 7240 in the memory 7230.

The screening/ diagnosis/ monitoring device 7200 may also contain a communication interface 7220. The code 7250 may contain instructions configured to allow the processor 7210 to communicate with an external computing device (not shown) via the communication interface 7220. The mode of communication may be wired or wireless. In one such implementation, the processor 7210 may transmit the stored RPG channel data from the data 7240 to the remote computing device. In such an implementation, the remote computing device may be configured to analyse the received RPG data to extract features. In another such implementation, the processor 7210 may transmit the analysis results from the data 7240 to the remote computing device.

Alternatively, if the memory 7230 is removable from the screening / diagnosis / monitoring device 7200, the remote computing device may be configured to be connected to the removable memory 7230. In such an implementation, the remote computing device may be configured to analyse the RPG data retrieved from the removable memory 7230 to extract the features.

### 4.9 RESPIRATORY THERAPY MODES

Various respiratory therapy modes may be implemented by the RPT device 4000. In some implementations of respiratory pressure therapy, the central controller 4230 sets the treatment pressure *Pt* according to the treatment pressure equation (1) as part of the therapy parameter determination algorithm 4329. $Pt = A Π \left(Φ, t\right) + {P}_{0}$ (1) where: *A* is the amplitude, Π(Φ, *t*) is the waveform template value (in the range 0 to 1) at the current value Φ of phase and t of time, and *P*₀ is a base pressure.

In one such implementation, the amplitude A is identically zero, so the treatment pressure *Pt* (which represents a target value to be achieved by the interface pressure *Pm* at the current instant of time) is identically equal to the base pressure *P*₀ throughout the respiratory cycle. Such implementations are generally grouped under the heading of CPAP therapy. In such implementations, there is no need for the therapy engine module 4320 to determine phase Φ or the waveform template Π(Φ).

In CPAP therapy, the base pressure *P*₀ may be a constant value that is hard-coded or manually entered to the RPT device 4000. Alternatively, the central controller 4230 may repeatedly compute the base pressure *P*₀ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

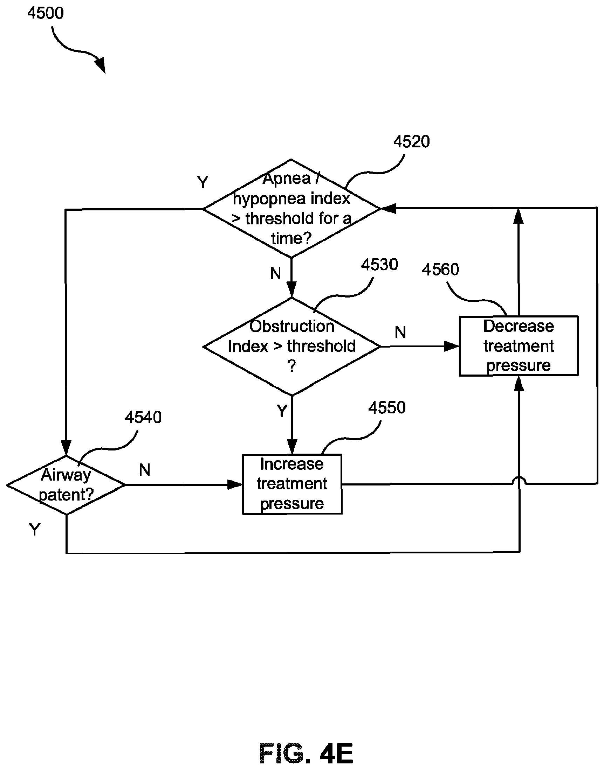

Fig. 4E is a flow chart illustrating a method 4500 carried out by the central controller 4230 to continuously compute the base pressure *P*₀ as part of an APAP therapy implementation of the therapy parameter determination algorithm 4329, when the pressure supportA is identically zero.

The method 4500 starts at step 4520, at which the central controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure *P*₀ by a predetermined pressure increment *ΔP,* provided the resulting treatment pressure *Pt* would not exceed a maximum *treatment pressure Pmax.* In one implementation, the predetermined pressure increment *ΔP* and maximum treatment pressure *Pmax* are 1 cmH₂O and 25 cmH₂O respectively. In other implementations, the pressure increment *ΔP* can be as low as 0.1 cmH₂O and as high as 3 cmH₂O, or as low as 0.5 cmH₂O and as high as 2 cmH₂O. In other implementations, the maximum treatment pressure *Pmax* can be as low as 15 cmH₂O and as high as 35 cmH₂O, or as low as 20 cmH₂O and as high as 30 cmH₂O. The method 4500 then returns to step 4520.

At step 4560, the central controller 4230 decreases the base pressure *P*₀ by a decrement, provided the decreased base pressure *P*₀ would not fall below a minimum treatment pressure *Pmin.* The method 4500 then returns to step 4520. In one implementation, the decrement is proportional to the value of *P*₀*-Pmin,* so that the decrease in *P*₀ to the minimum treatment pressure *Pmin* in the absence of any detected events is exponential. In one implementation, the constant of proportionality is set such that the time constant *τ* of the exponential decrease of *P*₀ is 60 minutes, and the minimum treatment pressure *Pmin* is 4 cmH₂O. In other implementations, the time constant *τ* could be as low as 1 minute and as high as 300 minutes, or as low as 5 minutes and as high as 180 minutes. In other implementations, the minimum treatment pressure *Pmin* can be as low as 0 cmH₂O and as high as 8 cmH₂O, or as low as 2 cmH₂O and as high as 6 cmH₂O. Alternatively, the decrement in *P*₀ could be predetermined, so the decrease in *P*₀ to the minimum treatment pressure *Pmin* in the absence of any detected events is linear.

In other implementations ofthis form ofthe present technology, the value of amplitude *A* in equation (1) may be positive. Such implementations are known as bi-level therapy, because in determining the treatment pressure *Pt* using equation (1) with positive amplitude A, the therapy parameter determination algorithm 4329 oscillates the treatment pressure Ptbetween two values or levels in synchrony with the spontaneous respiratory effort of the patient 1000. That is, based on the typical waveform templates Π(Φ, *t*) described above, the therapy parameter determination algorithm 4329 increases the treatment pressure *Pt* to *P*₀ *+ A* (known as the IPAP) at the start of, or during, or inspiration and decreases the treatment pressure *Pt* to the base pressure *P*₀ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude A, which has a "small" value (a few cmH₂O) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing retumed by the respective algorithms in the therapy engine module 4320 in analogous fashion to the computation of the base pressure *P*₀ in APAP therapy described above.

In other forms of bi-level therapy, the amplitude A is large enough that the RPT device 4000 does some or all of the work of breathing of the patient 1000. In such forms, known as pressure support ventilation therapy, the amplitude A is referred to as the pressure support, or swing. In pressure support ventilation therapy, the IPAP is the base pressure *P*₀ plus the pressure support A, and the EPAP is the base pressure *P*₀.

In some forms of pressure support ventilation therapy, known as fixed pressure support ventilation therapy, the pressure support *A* is fixed at a predetermined value, e.g. 10 cmH₂O. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the currentmeasure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure supportA so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support A is repeatedly computed as: $A = G \int \left(Vent-Vtgt\right) dt$ (2)
where G is the gain of the PI control. Larger values of gain G can result in positive feedback in the therapy engine module 4320. Smaller values of gain G may permit some residual untreated CSR or central sleep apnea. In some implementations, the gain G is fixed at a predetermined value, such as -0.4 cmH₂O/(L/min)/sec. Alternatively, the gain G may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations In yet other implementations, the gain G may vary depending on the difference between the current measure *Vent* of ventilation and the target ventilation *Vtgt.*

Other servo-ventilation control methodologies that may be applied by the therapy parameter determination algorithm 4329 include proportional (P), proportional-differential (PD), and proportional-integral-differential (PID).

The value of the pressure support A computed via equation **(Error! Reference source not found.)** may be clipped to a range defined as *[Amin, Amax].* In this implementation, the pressure support A sits by default at the minimum pressure support *Amin* until the measure of current ventilation *Vent falls* below the target ventilation *Vtgt,* at which point *A* starts increasing only falling back to *Amin* when *Vent* exceeds *Vtgt* once again.

The pressure *support limits Amin and Amax* are settings of the RPT device 4000, set for example by hard-coding during configuration of the RPT device 4000 or by manual entry through the input device 4220.

In pressure support ventilation therapy modes, the EPAP is the base pressure *P*₀. As with the base pressure *P*₀ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure *P*₀ in constant CPAP therapy.

Alternatively, the therapy parameter determination algorithm 4329 may repeatedly compute the base pressure *P*₀ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

In other forms of respiratory therapy, the pressure of the flow of air is not controlled as it is for respiratory pressure therapy. Rather, the central controller 4230 controls the pressure generator 4140 to deliver a flow of air whose device flow rate *Qd* is controlled to a treatment or target flow rate *Qtgt.* Such forms are generally grouped under the heading of flow therapy. In flow therapy, the treatment flow rate *Qtgt* may be a constant value that is hard-coded or manually entered to the RPT device 4000. If the treatment flow rate *Qtgt* is sufficient to exceed the patient's peak inspiratory flow rate, the therapy is generally referred to as high flow therapy (HFT). Alternatively, the treatment flow rate may be a profile *Qtgt*(*t*) that varies over the respiratory cycle.

### 4.10 DATA TRANSMISSION RESOLUTION

FIG. 1A shows a system 100 for monitoring the health of the patient 1000. The system 100 thus includes the respiratory pressure therapy device 4000, the humidifier 5000, an optional external device such as a mobile device 110, and a body mounted health monitoring device 120. The system 100 also includes a remote cloud-based health data analysis engine 130. The health data analysis engine 130 may run on a networked computing device or devices available through the cloud. The analysis engine 130 may be a cloud-based system in this example that is coupled via a network 140. The health analysis engine 130 thus is in network communication with the respiratory therapy device 4000.

The respiratory pressure therapy device 4000 includes a transmitter and an air control device to provide respiratory therapy to the patient 1000. As will be explained, the respiratory pressure therapy device 4000 collects operational data and transmits the collected operational data to the remote health data analysis engine 130. The health data analysis engine 130 receives the collected data from the respiratory therapy device 4000 to determine a health condition of the patient 1000 based on the collected data. The engine 130 may also receive and add other relevant data from a patient information database 150, the health monitoring device 120, and the mobile device 110. External databases, such as a database 160 may also provide additional data for the analysis of the health condition. For example, the database 160 may include "big data" from other respiratory pressure therapy devices and corresponding patients. The database 160 may also store relevant external data from other sources such as environmental data, scientific data, and demographic data. External devices such as a workstation 170, accessible by a health care provider, may be connected to the health analysis engine 130, as will be explained below.

Data from the database 150 and health conditions may be further correlated by a machine-learning engine 180. The machine-learning engine 180 may implement machine-learning structures such as a neural network, decision tree ensemble, support vector machine, Bayesian network, or gradient boosting machine. Such structures can be configured to implement either linear or non-linear predictive models for determining different health conditions. For example, data processing such as classification of a health condition may be carried out by any one or more of supervised machine learning, deep learning, a convolutional neural network, and a recurrent neural network. In addition to descriptive and predictive supervised machine learning with hand-crafted features, it is possible to implement deep learning on the machine-learning engine 180. This typically relies on a larger amount of scored (labelled) data (such as many hundreds of nights of scored sleep and health data from different RPT devices) for normal and abnormal conditions. This approach may implement many interconnected layers of neurons to form a neural network ("deeper" than a simple neural network), such that more and more complex features are "learned" by each layer. Machine learning can use many more variables than hand-crafted features or simple decision trees.

Convolutional neural networks (CNNs) are used widely in audio and image processing for inferring information (such as for face recognition), and can also be applied to audio spectrograms, or even population scale genomic data sets created from the collected data in the system 100 represented as images. When carrying outimage or spectrogram processing, the system cognitively "learns" temporal and frequency properties from intensity, spectral, and statistical estimates of the digitized image or spectrogram data.

In contrast to CNNs, not all problems can be represented as one with fixed-length inputs and outputs. For example, processing respiratory sounds or acoustic sounds of the heart has similarities with speech recognition and time series prediction. Thus the sound analysis can benefit from a system to store and use context information such as recurrent neural networks (RNNs) that can take the previous output or hidden states as inputs. In other words, they may be multilayered neural networks that can store information in context nodes. RNNs allow for processing of variable length inputs and outputs by maintaining state information across time steps, and may include LSTMs (long short term memories, types of "neurons" to enable RNNs increased control over, which can be unidirectional or bidirectional) to manage the vanishing gradient problem and/or by using gradient clipping.

The machine-learning engine 180 may be trained for supervised learning of known health conditions from known data inputs for assistance in analyzing input data. The machine-learning engine 180 may also be trained for unsupervised learning to determine unknown correlations between input data and health conditions, to increase the range of analysis of the health data analysis engine 130.

Data from additional sensors, such as those on a body-mounted health monitoring device 120 worn by the patient 1000, may be collected. The body-mounted health monitoring device 120 may be smart wearable clothing, smart watch, or a smart device, in order to capture data in a low impact manner continuously from the patient 1000. For example, the health monitoring device 120 may include one or more sensors such as an audio sensor, a heart rate sensor, a respiratory sensor, a ECG sensor, a photoplethysmography (PPG) sensor, an infrared sensor, an activity sensor, a radio frequency sensor, a SONAR sensor, an optical sensor, doppler radar motion sensors, a thermometer, or impedance, piezoelectric, photoelectric, or strain gauge type sensors. This data can be fused with other data sources collected during the day or data collected during certain periods of time, such as from operating the RPT device 4000. Data may be sent to the mobile computing device 110 that may be in communication with the RPT device 4000. Alternatively, data from the additional sensors on the health monitoring device 120 may be directly sent to the RPT device 4000. Data from the health monitoring device 120, RPT device 4000, or mobile computing device 110 may be transmitted to the cloud 140.

In this example, the RPT device 4000 may include electronic components to act as a communications hub to manage data transfer with other sensors in the vicinity of the patient 1000, and transfer of the collected data for remote processing by the health analysis engine 130. Example of other sensors may include blood pressure monitors, weighing scales, sleep sensors, blood glucose monitors, and smart drug/medication adherence bins. Such data may be collected by the RPT device 4000 even when the RPT device 4000 is not delivering therapy itself. Alternatively, the mobile device 110 may collect data from the health monitoring device 120, the RPT device 4000, and other data sources, and thus serve as a communications hub to manage data transferto remote processingto the health analysis engine 130. Other devices such as home digital assistants that may communicate with the RPT device 4000 may also serve as the communications hub.

The example RPT device 4000 includes integrated sensors and communication electronics, as shown in FIG. 4C. Older RPT devices may be retrofitted with a sensor module that may include communication electronics for transmitting collected data. Such a sensor module could be attached to the RPT device and thus transmit operational data to the remote analysis engine 130.

"Integrated care" is a term that reflects a concern to improve patient experience and achieve greater efficiency and value from health delivery systems. The example system 100 in FIG. 1A relates to providing a complete solution for the patient, the health care provider, and the payor, incorporating high data rate and/or smart sensing and analytics from data from respiratory therapy devices.

In this example, the system 100 may change the "resolution of data" that is uploaded from a PAP device such as the RPT device 4000 to the cloud 140, the edge of the cloud, etc., based on an event and/or for a specific purpose that requires greater detail in the collected data. These smart health insights into co-morbidities with high resolution data and sensing can be enabled by utilizing the contact the RPT device 4000 has with the patient 1000 throughout the night (mask/pillows). This allows a change from traditional low resolution breath analysis to high resolution cardio-respiratory insights, augmented by cloud processing from the health analysis engine 130.

In this example, the RPT device 4000 may output air flow and pressure signals at a relatively low frequency, such as 25 Hz. Other low-resolution data such as mask pressure, air pressure, EPR pressure, leak data, respiratory rate, tidal volume, minute ventilation, snoring and flow limitation may be collected every two (2) seconds. Optional external sensors that may be connected to the RPT device 4000 may allow other data such as oxygenation (SpO2) and pulse rate to be collected. In addition, standard annotations for exchange and storage of medical data, such as the European Data Format (EDF), may be applied to the collected data. As will be explained, the above data may be collected at a higher resolution mode. In addition, the high resolution mode may allow the collection of additional types of data or data derived from the basic data.

The example health monitoring system 100 is based on the example RPT device 4000 serving as a connected hub in the home environment. The hub role is combined with the external health data analysis engine 130 for managing both sleep-disordered breathing and chronic disease in this example. This could be offered as care as a service to an integrated payor. The RPT device 4000 and associated sensors such as the health monitoring device 120 can monitor all aspects of chronic disease that the patient 1000 may be suffering from. For example, the analysis engine 130 may predict exacerbations (e.g., Asthma attack, COPD exacerbation) or cardiac conditions (Paroxysmal atrial fibrillation, worsening Atrial fibrillation, or Stroke) before they occur, based on triggering events that may be determined from the collected data. Other diseases and health conditions may be monitored based on the correlation of the collected data. Such correlations may be determined by the machine-learning engine 180 in FIG. 1A.

In this example, there are different triggering events that may result in an increase in the rate and type of data collection from the RPT device 4000. Thus, the RPT device 4000 may include two data collection modes. A low rate collection mode may include collection of standard operational data. A high rate collection mode may increase the collection rate and/or types of data that may be used for analyzing health conditions over a predetermined period of time in comparison to the low rate collection mode. The process forthe health monitoring system is based on the triggering event determined from data collected at the low rate collection mode. A determination is made as to whether a triggering event occurs, and what the event signifies.

After a triggering event is detected, there can be a determination of whether the triggering event was real. Verification of a real triggering event can be based on data from multiple sensors or duration of the event. If the determination is made that the triggering event was not real, the collection of data reverts back to the level of data collection before the triggering event. Optionally, the devices involved in the triggering event can perform diagnostics to determine if there is an issue/error that generated the false event. If the triggering reflects a real event, the processing can proceed based on data collection at a different mode.

The data resolution that changes can be a higher data rate that is sampled by the device and uploaded to the cloud or a higher data rate that is uploaded to the cloud but already sampled by the device. The sampling may be made from the sensors such as those shown in FIG. 4C. These may include sensors in the mask 3000, and flow sensors and acoustic sensors in the respiratory pressure therapy device 4000. Additional sensors can startup/shutdown to control (increase/decrease) the amount of data and/or data rate.

In the event of a real triggering event, a severity check can occur to determine the urgency or severity required of responsive action. The severity determination is based on how severe the triggering event may be. For example, a low severity event may trigger a notice to the patient to take action. A high severity event may require action taken by clinician or a call for emergency response. The determination of the triggering event and the evaluation of the severity of the event may be based on rules or controlled automatically by a machine-learning algorithm. The machine-learning engine 180 may be taught to determine different triggering events based on a training set of collected input data and triggering events. Similarly, the machine-learning engine 180 may be taught to determine the severity of different triggering events and the appropriate response. The weights to determine both a triggering event and the level of response may be refined by the machine-learning engine 180 as additional data is collected, and an evaluation of the resulting outcomes is made.

For example, one triggering event may be a request to build up health records for the user or patient that may be initiated by the patient 1000 or a caregiver. Such a command may be provided remotely via the mobile device 110 or a remote external device such as the workstation 170 in FIG. 1A. For example, the request to build up a health record may be made to establish a new baseline for the user, detect normal trends of the user to compare to longitudinal data in the future, or a request to access electronic health records. Alternatively, the baseline may be determined by adding the data from the patient to a baseline relating to a patient population of normative values for the type of patient.

The collected data for such requests may include cardiac information such as heart rate values, averaged (such as running mean or median) heart rate, a baseline heart rate, trends in heart rate over different time scales, heart rate variability (changes in interbeat interval) over different time scales. Different timescales could be on a second, minute, hour, multiple hours, day, or other intervals. Heart rate values (when combined with PAP and non-PAP sensing) may also be related to day or night times, whether the user is asleep, and in what sleep stage, whether the user is sitting quietly, exercising, and any other relevant factors. Cardiac output and CO2 levels can also be monitored over time. An analysis of cardiogenic oscillations can be used to infer the health of the lungs (and heart) over time, by giving insight into regional gas flow and distribution within the lungs. Similarly, the data could include movement metrics that are processed to estimate activity over different timescales and processing, such as movement of the user in bed with using PAP, or not using PAP, or during daily life. These data could be processed to calculate energy expenditure of the patient.

Respiration data can be processed to track inspiration and expiration values, to estimate respiration rate, determine any apneas or hypopneas, determine snoring duration, intensity and type, and trends over different timescales. Machine parameters, such as mask leak or vent leak, can be tracked overtime as will be explained below. Gas content in expired breath can be analyzed to detect unusual content, as well as an analysis of chemicals in the environment (e.g., tobacco smoke, scented laundry detergent, etc.). An estimate of dyspnea can be generated based on breathing rate, breathing curve (indicate of faster and shallower breathing), cardiac changes, such as palpitations and potentially change in cardiac output, and may include user subjective feedback on a feeling of difficulty breathing or feeling smothered. Changes in sleep architecture, such as an increase in wake time, and/or more fragmented sleep (number of arousal or awakenings, and duration of each), can also be captured. Any of these parameters may be compared to global or personalized trigger levels.

Another example of a triggering event is an unusual lack of using an RPT device. The interruption of low rate data collection from the example RPT device 4000 may alert a health care provider or patient that the patient is away from home and has not brought a travel device with them. Another situation may be if the patient is too sick to operate the therapy device. In such a case, other living space sensors such as a smart assistant, mattress or bed sensor, light sensor, temperature sensor, occupancy sensor, passive infrared (PIR) sensor, video/security camera, radio frequency (RF) imaging sensor (such as UWB or IR-UWB), microwave sensor, building or home smart management system, intruder alarm sensors and setting (current mode such as enabled or disabled), smart meter (measuring electricity usage), door operated switch, audio detection sensor, fall detection sensor, presence of a smartphone and/or Bluetooth beacon, smartphone battery/charge/usage including user interface (UI) interactions or movements, may be accessed to check the condition of the patient.

Another example scenario may be if a patient has forgotten to operate their therapy device. A push notification may be sent to a smart device such as the mobile device 110 to remind the patient. Another example scenario may be if a consumable such as a filter, oxygen tank, or a battery has been depleted, preventing use of the device. The system 100 may then provide communication to a supply system to drop-ship a replacement consumable directly to the patient, or provide delivery of the consumable to a pharmacy for patient pickup or to a technician for installation at the patient location.

According to the claimed invention, triggering enhanced data collection is the provision of new medication or treatment. For example, if new medication is provided either new to the user or new to the market where the user is embarking on a new or changed drug regime, the system 100 can monitor breathing and cardiac activity at a higher collection rate (and potentially carry out gas analysis) to check that the new treatment is successful and does not have side effects or interactions with other medications. Key data may include changes in heart condition, such as changes in heart rate over time (and changes in heart rate by sleep stage), changes in heart rate variability (e.g., greater variability may be a sign of improvement), reduction in paroxysmal atrial fibrillation, increase in cardiac output, changes in blood pressure, changes in breathing rate, changes in breathing depth, changes in activity level, reduction in estimated discomfort, increase in sleep quality, reduction in insomnia symptoms, increase in reported energy levels, reduction in residual apnea or snoring or wheezing or coughing, improvement in lung functioning, reduction in PAP pressure required, increase in usage of PAP, and reduction in reported depression or anxiety. Insomnia is a complaint of dissatisfaction with sleep quality or duration, involving difficulties in initiating sleep at bedtime, frequent or prolonged awakenings, or early-morning awakening with inability to return to sleep. In addition, it can include significant day time distress, impaired functioning, mood disturbance, reduced cognition, and fatigue.

A similar procedure may be used to evaluate medication or treatment in general for the patient. The medication amount or frequency of dosing may be adjusted automatically if the patient has medication delivery via a platform integrated with the RPT device 4000. Such a delivery platform may include a drug reservoir. For example, the drug reservoir may be used in conjunction with a patch for daytime delivery (thus saving power and medication contained in the patch. The RPT device 4000 may thus control the drug reservoir to administer the medication while the RPT device 4000 is in use (typically at night). Thus, a platform for medication delivery is realized with routine delivery of medication or exceptional delivery of medication based on a triggering event. Based on clinical review and approval, certain medications such as bronchodilators, anti-inflammatories, and antibiotics may be delivered to the patient. These may be delivered while the patient is awake and wearing a patient interface. Delivery of such medications may be made by pressing a button. This delivery may be combined with oxygen delivery, for example. For a person that is detected as having an asthma attack, instead of the RPT device 4000 increasing the pressure (which could worsen the situation), the system 100 can offer the patient the ability to request (or automatically) deliver a relief dosage via the patient interface or the mobile device 110, and activate a breathing program to help calm the patient. The mobile device 110 may include an application that communicates additional instructions to the patient. For example, an application may also ask the patient to sit upright and may contact emergency services or another health care provider. Similarly, medication may be offered for patients having (or predicated to have) COPD exacerbations or CHF decompensations. Other medications, such as nasal decongestants, may treat an upper airway infection related to the common cold or influenza (e.g., to try to prevent this deteriorating into an exacerbation). Other devices that administer medication may be communicatively integrated with the RPT device 4000. For example, medication may be delivered via a smart inhaler, a combination monitor accessory attached to an inhaler such as those offered by Propeller Health, or other smart-connected metered medication delivery system/devices, that may communicate the occurrence of dosages of medication inhaled by the patient 1000 and other data.

The enhanced data may be used to analyze different elements of respiratory pressure therapy, such as whether the condition of the patient is changing, or satisfying a threshold or trend. For example, the air flow data may be used to determine how a CPAP device is adapting to the patient. This data may be used to determine whether sleep-disordered breathing is worsening or improving over time. For example, an open or closed patient airway can be detected by inducing airflow with a CPAP device and producing a pressure modulation. Demodulating the measured airflow signal from the flow sensor 4274 separates this modulating signal from heartbeat and other factors. An airway open is indicated if the mean induced signal is more than 0.03 l/sec and an airway closed is indicated if the mean induced signal is less than 0.03 l/sec. Other data may be collected to estimate cardiovascular fitness or to determine respiratory rates, hydration, Ischemia, blood pressure, and cholesterol levels of the patient 1000.

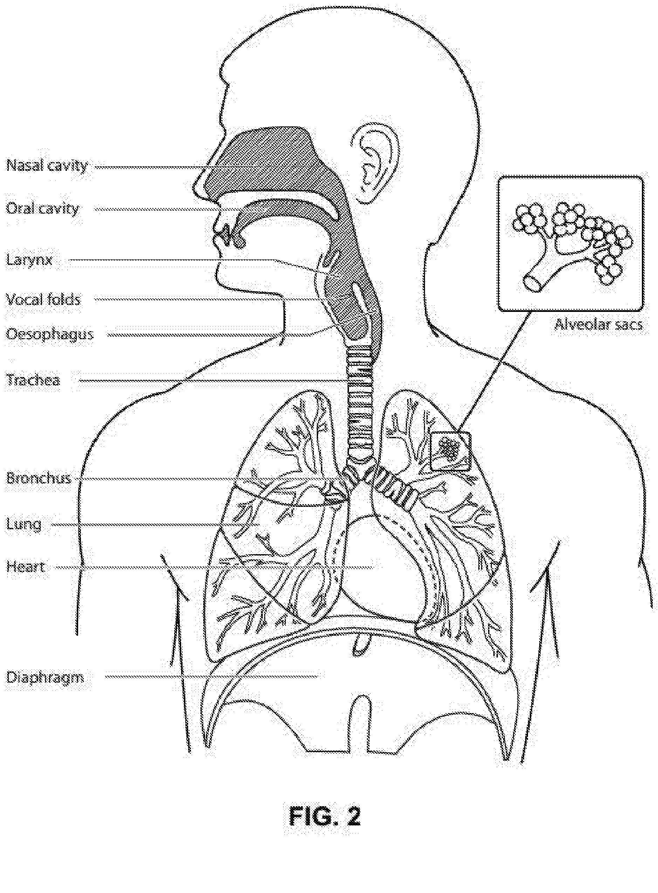

The flow data, motor speed, pressure and acoustic data from the example RPT device 4000 may be used to profile the characteristics of the RPT device 4000. As explained above, the sensors may be used to detect the flow generation from the motor, characteristics from the respiratory apparatus conduit such as the tube or hose to the mask, the mask or cannula fit, motor speed, and gas volumetric flow rate and outlet pressure (from a pressure transducer or a flow sensor). For example, this data may be used to identify accessories and parts such as mask type, tube or hose, number of hoses, and connectors. Data may be collected to determine any leaks that are indicative if the fit of the mask interface 3000 is within acceptable parameters. This may also be correlated to whether a patient is using a mask of the best size or type, or whether the mask specifically must be replaced due to ordinary wear and tear. The tube may act as an acoustic waveguide from the internal acoustic sensor 4278 in FIG. 2C. Data may be collected by the flow generator being run at a constant speed (to have largely flat sound data signal) or have a change introduced at a specific time.

The raw acoustic data from the acoustic sensor 4278 may be processed in order to determine the transfer function ofthe tube, mask, and respiratory system to gain new insights about the condition of the equipment, the lungs of the patient 1000, and gas composition. The transfer function may also be used to detect patient movement. For example, the reflection from the motor sound may show a peak relating to the round trip time from the acoustic sensor 4278 to the mask cavity, and back. The system has knowledge of the tube length, and the speed of sound for various gases (such as typical composition of air, inhaled air, and changed composition when exhaled carbon dioxide is greater than the base CO2 level in air, or indeed any expected changes due to supplemental oxygen, changes in speed of sound of different gases at different temperatures, and humidity settings).

One approach to model the transfer function by calculating the impulse response function is using "Cepstrum" analysis. Cepstrum analysis has origins in analyzing seismic events and is useful in processing echo signals. Cepstrum analysis can allow separating different effects, as different events can be additive in the logarithm of the power spectrum and separable. Other processing approaches could include using a logarithmic power spectrum (LogFFT), a Segmented Chirp Z-Transform (SCZT) together with a flexible window, or time-frequency representations (TFRs) such the Short-Time Fourier Transform (STFT), Gabor expansion, the Cross-Ambiguity Function (CAF), and quadratic TFRs such as the Wigner-Ville Distribution (WVD). When considering Cepstral analysis, the autocepstrum is the ceptstrum of the autocorrelation of the signal. Cepstrum analysis involves the processing of sound samples from the tube, carrying out a Fourier transform, taking the Logarithm, and then an Inverse Fourier transform. The purpose of the process is to convert the convolution of an impulse response function (IRF) and a noise or sound signal into an addition operation to more easily allow the separation of the IRF for analysis.

For example, to detect a kink or obstruction in the tube, a reference value could be calculated initially (e.g., a training sample of an unobstructed tube), and then a new measurement taken when the tube is in operation (e.g., where the tube has gotten kinked, causing an obstruction), and calculate the difference (e.g., of a significant sample of this difference) to estimate the location (in the tube) by determining the location of a "significant" sample such as a peak. The extent or severity of the obstruction may be determined from the change in peak amplitude, or profile. Alternatively, auto correlation (inverse Fourier transform of the power spectrum) may be used. In a similar way, the mask type, size, and person's respiratory system can be modelled based on analysis of the IRF, such as using deep learning imaging/spectrogram methods (or directly, based on processing of the received audio samples).

The collected data may also be used to determine an analysis of sleep stage. For example, the movement of the patient interface, such as the mask 3000, may be determined from an embedded motion sensor to determine movement of the patient. Normalized respiration rate variability and inspiration/expiration ratio changes may be determined for sleep stage analysis. Heart rate variability trends may also be determined for sleep stage analysis. For example, by tracking peaks in the cardiogenic signal seen in one or more of the flow, pressure, or microphone signals, the peak-to-peak time can be used to estimate number of heart beats per minute (HR bpm) and well as heart rate variability (such as spectral analysis of interbeat times). Such data may be correlated to how sleep architecture is evolving and used to determine whether the patient is proceeding through the expected number of sleep stages, whether the patient is sleep fragmented, whether the patient is receiving adequate REM and deep sleep, and whether the patient is going to the toilet for frequent urination. As inputs to a machine learning model, features such as age and gender information may be used. Estimated movement intensity, duration, and patterns of activity, heart rate, heart rate variability, breathing rate, breathing rate variability, normalized breathing rate variability, breathing signal waveform shape (inspiration, expiration pauses) may be calculated as part of sleep staging analysis system. In some embodiments, the flow and/or microphone signals may be supplied directly to the model, such as deep learning neural network system. The system may calculate wakefulness (conscious and unconscious micro-awakenings), light sleep (stages NREM N1, N2), N3 deep sleep / slow wave sleep, and REM sleep. The system learns changes in breathing rate and or heart rate variability during different stages of sleep, and the relationship with movements of the patient. Knowledge of sleep stage patterns can be used to adapt the therapy settings in order to optimize deep and REM sleep, maximize overall sleep time, and reduce awakenings and light sleep. Demographic data such as age and gender information for may be input into a machine learning model to determine the sleep stage. Inputs may include age, gender, estimated movement intensity, duration, and patterns of activity, heart rate, heart rate variability, breathing rate, breathing rate variability, normalized breathing rate variability, and breathing signal waveform shape (inspiration, expiration pauses) to a sleep staging analysis blocks for the machine learning model.

The sleep stage analysis may also be combined with other data to determine stress, mental health issues, or other physiological issues. For example, during a sleep stage, a drop in heart rate and blood pressure may be expected. If the collected data indicates a deviation from the expected drops, this provides an indication of stress, mental health issues, or other physiological issues. Another example may be predicting the likelihood of complex sleep apnea before commencing continuous positive airway pressure. This can be related to the severity of diagnosed sleep-disordered breathing before treatment, the prevalence of central events during that test, and continuing monitoring of central and obstructive events (and examples of periodic breathing) after diagnoses but prior to treatment (such as via acoustic or RF sensing or other movement and breathing estimator of sleep). Analysis of the sleep hypnogram/sleep architecture, particularly disruption/fragmentation, and the ratio of the hypopnea density in NREM versus REM sleep can also be used as an input feature. Appropriatetherapy may then be selected to address the condition.

The collected data may be used to determine respiration changes for the tracking of changes in conditions, such as COPD (e.g., higher than normal breathing rate/tachypnea). The collection of respiration data overtime may determine how base (e.g., an average "baseline" level) respiration rate evolves over time. The collection of respiration data may also be used to measure expiration, expiratory pressure relief (EPR) back-off time during expiration (e.g., as configured and delivered by the RPT), overall inspiration time and amplitude, and breath-hold time (time before next cycle). Such disease analysis may include tracking worsening disease conditions. The collected data may be analyzed to detect worsening Asthma, pollen allergy, common cold, or respiratory infections. For example, lung impedance may be monitored to detect changes in lung condition over time. Other increases in patient airway resistance may be indicative of the worsening conditions. For example, COPD and Asthma are diseases in which airway narrowing occurs and thus exhibit an increase in airway resistance, which can be detected or estimated.

The data from a sensing device such as a spirometer (or a similar apparatus combined with or enabled by the RPT) may measure the respiratory flow rate, and calculate the inspiratory and expiratory lung volume. This data may be correlated with blood chemistry in addition to data collected from devices such as a glucose monitor or gas detection of volatile organic compounds (VOCs) produced by the body's metabolic activity; these gaseous molecules can be detected from breath. For example, certain VOCs have been associated with COPD, Asthma, and CHF. Thus, the data may determine detected biomarkers that are suggestive of CHF, Diabetes, other chronic conditions. Blood glucose may be determined from sampling breath throughout the night. For example, a volatile organic compound sensor may be added to the RPT 4000 to monitor diabetes. The VOC sensor may also be used to monitorthe endocrine system by sampling signals to measure gas content (including CO2) to understand hormonal balance during sleep. The sleep stage may be checked against hormone production via gas analysis, respiration, cardiac, and movement parameters determined from the collected data.

The collected data may also be analyzed to determine other health events that are not directly related to respiratory conditions. For example, data may be correlated to changes in heart rate, cardiac output, and cardiac fiducial signals. The heart rate may be determined by pressure modulation due to heart beat arising from cardiogenic oscillations. Data may be correlated to determine whether the heart is operating effectively as a pump, whether heart rate drops as expected when asleep, level or respiratory sinus arrhythmia, signs of bradycardia, tachycardia, premature atrial contractions (PACs), premature ventricular contractions (PVCs), paroxsysmal atrial fibrillation (PAF), atrial fibrillation (AF), atrial flutter, ventricular tachycardia, and ventricular fibrillation. Certain conditions such as ventricular fibrillation may cause an alert (e.g, medical emergency) to be generated or cause therapy to be applied. Additional correlations for non-respiratory conditions may be determined by collected data from numerous patients via the machine-learning engine 180.

Audio data may be used to confirm or enhance health condition analysis. For example, sound data of breathing from a patient from the internal audio sensor 4278 in FIG. 4C may be used in conjunction with external sounds detected by the external audio sensor 4279 to determine sound data. In order to collected detailed audio data, the RPT 4000 may be controlled to step down or stop the blower to obtain a sufficient audio sample. One example of sound data processing may be the Cepstrum analysis explained above.

The sound data may include the level of residual snoring, gasping, wheezing spluttering, and the sound of the heartbeat. These sounds may be used to determine respiratory and other health conditions. For example, the intensity and timing (inspiration or expiration) of a wheeze sound may be a symptom of respiratory conditions, disorders, or ailments. In addition, lack of sounds, such as a silent chest, may indicate severe asthma in combination with other vital signs like a higher heart rate and respiration rate.

Cardiac insights may be obtained through acoustic processing from data from the internal and external acoustic sensors 4278 and 4279 in FIG. 4C. This data may be combined with the use of the mask and airflow data to monitor cardiac output and heart rate. The sound data may be used to model the tube, mask, and upper airway (nose, pharynx, and larynx) and the lower airway (trachea, bronchial tubes, and alveoli).

The collected data may be analyzed in the context of specific patient conditions that may be derived from data from other sources. Such data may include data input through an application running on the mobile device 110, or input from electronic health records on a database such as the database 160 in FIG. 1A. The patient-specific data may therefore include conditions a patient may have such as pre-existing issues, demographic details (BMI, age, gender), and geographic details (allergen risks due to pollen count, heat exhaustion due to outside temperature, air quality and oxygen quantity due to altitude), and medications associated with the patient.

The patient input data may include subjective feedback on how the patient is feeling whether the patient feels fatigued, and the level of sleepiness. The data may be used to determine the quality of sleep for the patient. This may be a comparison to a personal baseline forthe patient, linked to weather data, a comparison to an average sleeper of their age and gender (aiming to be better than average), or a comparison to an average of a person with the same chronic conditions and or disease progression. As explained above, the baseline may be one determined from a normative patient population relative to the patient. Such data may be displayed in an application executed by the mobile device 110. Such data may also be made available on a work station 170 for a health care professional.

A sensor on the RPT device 4000 may sense gas (breath) from the patient 1000 using for example an array of cross-reactive sensors, and pattern recognition/deep learning to identify the characteristic changes in certain VOCs due to disease progression. Additional sensors may detect gases, fumes, smoke, or particulates, in a room environment where the RPT device 4000 is located as well in the exhaled gas of the patient (such as measuring the quantity of PM2.5 (inhalable particles with a diameter of generally 2.5 micrometers and smaller) and PM10 (particles with a diameter of 10 micrometers and smaller). Such data may be used to determine whether bacteria, viruses, and other contaminants have been adequately filtered, such as via a HEPA filter removing 0.3 micron particles and smaller, which includes most mold, mildew, and viruses.

As explained above, another device such as a smartphone, smart device, smart speakers such as Alexa or Google Home, smart services such as Siri or Bixby may include an application that may trigger the increased collection of data. For example, acoustic sensing may run on the device to listen for breath sounds, such as inspiration, expiration, coughing, wheezing, snuffling sneezing, gasping, and whistle. Acoustic sensing may also be active using SONAR to sense a reflected signal from the chest. An RF sensor may be integrated into a smart device to detect ballistocardiogram (movement or heart), movement of the chest and abdomen with breathing, or activity.

A processing event may be triggered if a new sensor or other data streams become available. This could include allocating more processing resources to analyze more detailed waveforms (such as requiring higher data transfer, and potentially higher energy consumption) for a period of time to identify and manage a new or worsening condition. In addition, a new device (such as a new CPAP) may trigger the increased collection of data. The increased data may allow a change in device, calibration to new settings, and configuration with an optimized profile of the user. The increased data may be used to confirm that the RPT device 4000 (including the motor) is functioning as expected. For example, filters may be determined to be clean based on the processing of data from the internal acoustic sensor 4278.

For example, one event may be if ECG data is derived from the collected data that shows signs of left ventricular hypertrophy, presence of atrial fibrillation (irregularly irregular beats), or tachycardia. The system may then determine the events signify a possible stroke. Such data may also signify a heart attack, stress (heart rate averages do not reduce as expected during sleep), COPD, or complex apnea (increase in central events seen before and during early therapy).

Such triggering events may also indicate incorrect RPT device settings, an uncompliant user, blood pressure, asthma attack, and arrhythmias such as diabetes. Generally, an outcome is then determined by the analysis. The patient is notified, and the patient may take a recommended action. For example, the actions may include actions that may be communicated to the patient via the mobile device 110. For example, a message may be sent to the patient to check a bio-signal related to the event. The actions may also include a notification that indicates that medication is required, which the patient may already have in their possession. The event may be to prompt the patient for a current feeling or health condition. For example, the application on the mobile device 110 may ask a patient to input responses to inquiries such as "how do you feel" or other questions in relation to the current state of the patient. Such data may be collected from patient input to the application in the form of a slider or a numerical rating. Such subjective information may provide additional verification to cross-check collected objective data streams. The responses of the patient may therefore be used as a feedback mechanism to reduce risk of a false positive triggering event. The responses may also be compared to previous patient inputs. A change in one or some of these user reported issues (and increase in perceived quality of life such as increased alertness, ability to walk, or walk a longer distance, not feeling breathless, or clearing up a respiratory infection), along with an improvement in the analysis of the collected data may be captured by the system. An application on the mobile device 100 may also suggest that the patient consult a health care professional. The application may give the patient the underlying data generated from the health analysis engine 130. The application may provide the data to indicate good health, which can be used for rewards or incentives such as a health insurance discount. The patient may be provided coaching based on the granular data.

In other examples, other actors such as payors may take action in response to a triggering event. For example, the payor could be a government official, employer, insurance provider, or doctor. Information may allow the payor to request the patient come to a health care facility for new tests, as the information may indicate a new and/or unknown/undiagnosed condition. The payor may determine the onset of disease related to sleep or not and avoid readmission of the patient. Clinician specific analysis may allow a health care provider to focus on which patients to insure/treat, and track the efficacy of medications/treatments.

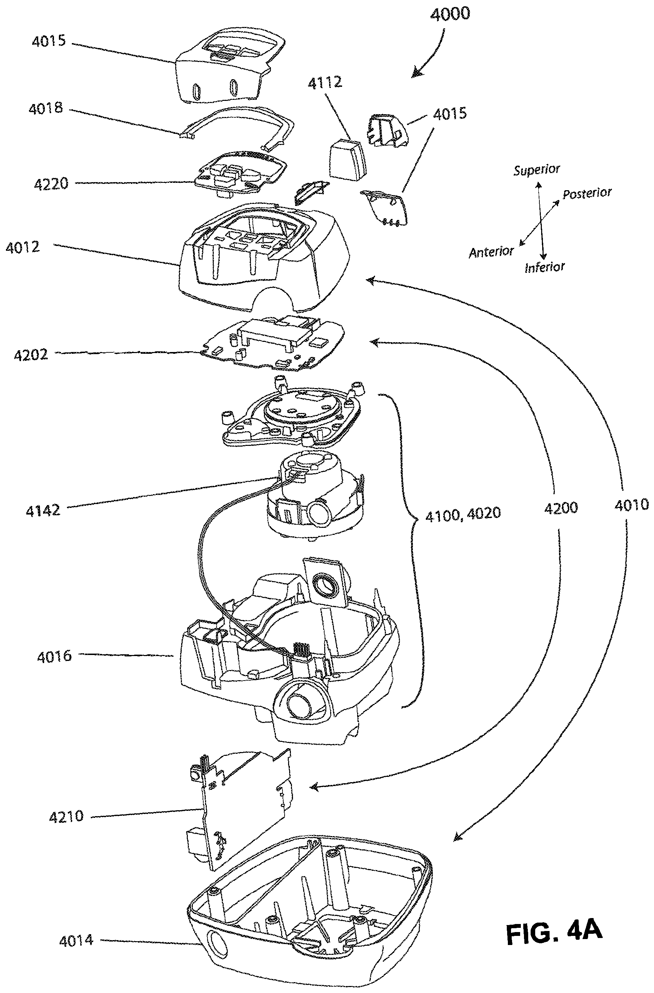

Connected devices such as the RPT 4000 in FIG. 4A are capable of storing and sending varying levels of data. For example, the central controller 4230 in FIG. 4C may send data to the external source 4286. Such data may include the data gathered by the sensors of the RPT 4000, such as the flow rate sensor 4272 or pressure sensor 4272, data generated by the algorithms of the pre-processing module 4310, or data generated by the algorithms of the therapy engine module 4320. Alternatively, devices such as the headbox 2000 in FIG. 7A may include sensors as described above that may provide additional data. Such data may be combined for analysis by algorithms that generate yet more data.

Such data may be segregated into low resolution data that is transmitted externally in most normal operation conditions of the RPT 4000 and high resolution data that is transmitted in certain exceptional circumstances. In this example, low resolution data may be considered as data that is sampled once per minute or less by the controller 4230 in FIG. 4C. High resolution data may be defined as data that is sampled more than once per minute. In such an example, low and high resolution data is the same type of sensed data, differing only in rate that that data is sampled from the patient. Of course, high resolution data may be different types of sensed data from that of low resolution data. In such a case, the high resolution data is types of data not ordinarily transmitted to external devices by the central controller 4230. Of course, high resolution data may also be a combination of low resolution data at a higher sample rate and additional types of data different from low resolution data.

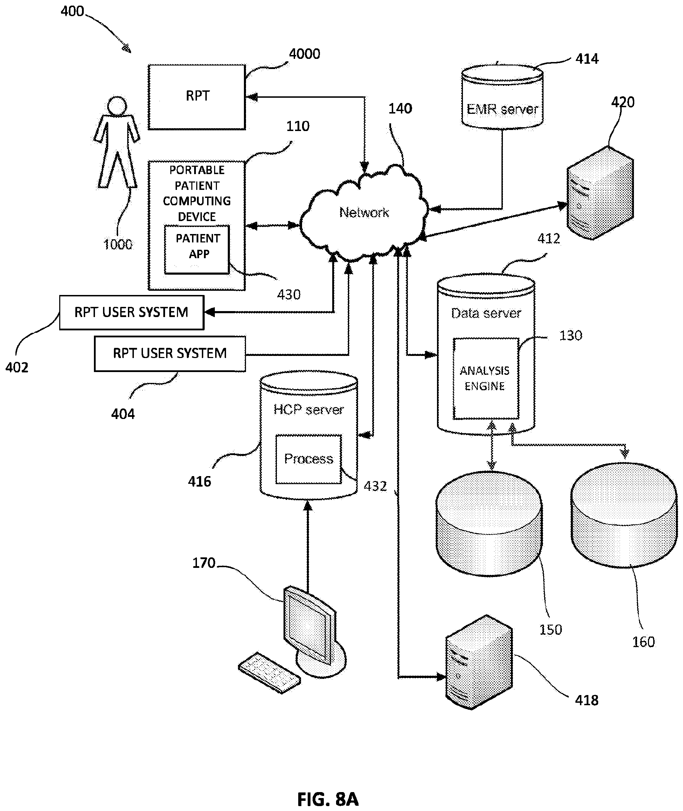

FIG. 8A shows a block diagram of an example health care system 400 that collects operational data from RPT devices such as the RPT device 4000 used by the patient 1000 in FIG. 1 and provides health care services to the patient 1000. The health care system 400 may include multiple patients. Each of the patients may be using an RPT device with functionality similar to the RPT device 4000, additional sensors, such as the health monitoring device 120, and associated mobile computing devices. Collectively, the RPT device, sensors, and mobile computing device for different patients may be shown as RPT systems 402 and 404. Such systems collect data from the corresponding patients requiring respiratory therapy for the health care system 400. The health care system 400 includes a data server 412, an electronic medical records (EMR) server 414, a health or home care provider (HCP) server 416, a supply and support server 418, and a payor data server 420. The data server 412 executes the health care analysis engine 130 and has access to different databases, such as the patient information database 150 and other databases such as the database 160. In the system 400, these entities are all connected to, and configured to communicate with each other over the wide area network 140, such as the cloud or Internet. The connections to the wide area network 140 may be wired or wireless. The data server 412, EMR server 414, the HCP server 416, the support server 418, and the payor server 420 may all be implemented on distinct computing devices at separate locations, or any sub-combination of two or more of those entities may be co-implemented on the same computing device.

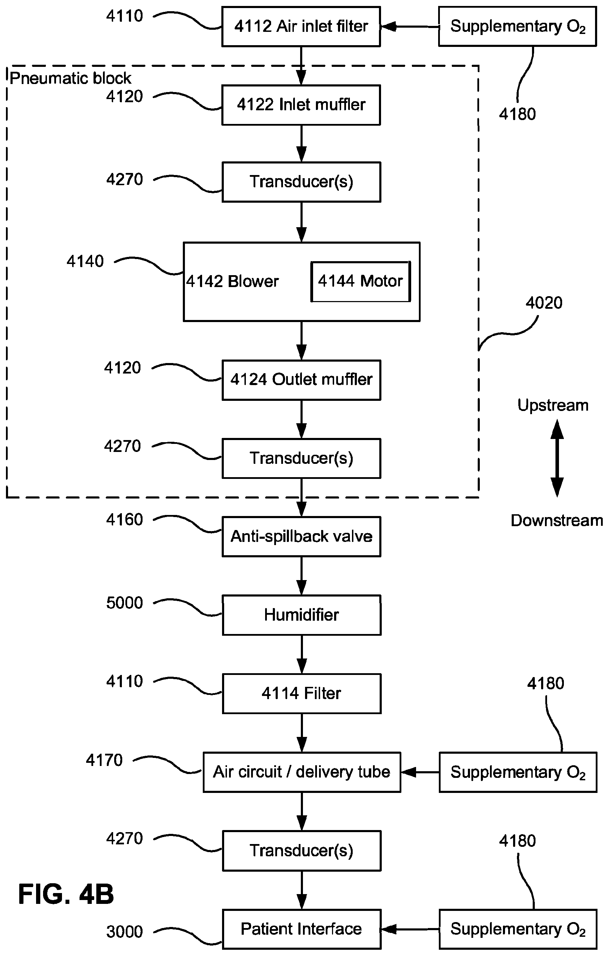

In this example, either the RPT 4000 or the mobile device 110 associated with the patient is configured to intermediate between the patient 1000 and the remotely located entities of the system 400 over the wide area network 140. In the implementation of FIG. 4, this intermediation is accomplished by a software application program 430 that runs on the mobile device 110 or on the RPT 4000. Such an application may be a dedicated application or a web browser that interacts with a website provided by the health or home care provider.

As explained above, the collected data from the RPT 4000 may be provided for patient health condition analysis of the patient 1000 conducted by the health care analysis engine 130. The analysis may also include monitoring of the application of treatment or medication for the particular patient 1000 as explained above. In this example, the RPT 4000 or the mobile device 110 are configured to transmit the data collected from the RPT 4000 and other devices via a wireless protocol, which receives the data as part of the application run by the respective controllers. The RPT 4000 or the mobile device 110 transmits the data to the data server 412 according to "pull or push" model. The data server 412 may receive the data according to a "pull" model whereby the RPT 4000 transmits operational data in response to a query from the data server 412. Alternatively, the data server 412 may receive the collected data according to a "push" model whereby the RPT device 4000 transmits the event data to the data server 412 as soon as it is available. Further, the data server 412 may access databases 150 to store collected and analyzed data relating to the patient 1000 and other databases 160 for big data relating to overall populations of patients or other relevant data.

Collected data received from the RPT device 4000 or the mobile device 110 is stored and indexed in the database 150 by the data server 412 so as to be uniquely associated with the patient 1000 and therefore distinguishable from data collected from other devices in the system 400. In this regard, although only three RPT user systems are illustrated in FIG. 4 for ease of explanation, the system 400 may include many more RPT user systems with corresponding RPT devices, sensors, mobile computing devices, and other components. The data server 412 may be configured to calculate summary data for each session from the data received from the RPT device 4000. The data server 412 may also be configured to receive data from the mobile devices 110, including data entered by the respective patient 1000, behavioral data about the patient, or qualitative data.

The EMR server 414 contains electronic medical records (EMRs), both specific to the patients of the system 400 and generic to a larger population of patients with similar disorders to the patient 1000. An EMR, sometimes referred to as an electronic health record (EHR), typically contains a medical history of a patient, including previous conditions, treatments, co-morbidities, and current status. The EMR server 414 may be located, for example, at a hospital where any of the patient 1000 has previously received treatment. The EMR server 414 is configured to transmit EMR data to the data server 412, possibly in response to a query received from the data server 412.

In this example, the HCP server 416 is associated with the health/home care provider (which may be an individual health care professional or an organization) that is responsible for the patient's respiratory therapy. An HCP may also be referred to as a DME or HME (domestic/home medical equipment provider). The HCP server 416 may host a process 432 that is described in more detail below. One function of the HCP server process 432 is to transmit data relating to the patients to the data server 412, possibly in response to a query received from the data server 412.

In some implementations, the data server 412 is configured to communicate with the HCP server 416 to trigger notifications or action recommendations to an agent of the HCP such as a nurse, or to support reporting of various kinds. Details of actions carried out are stored by the data server 412 as part of the engagement data. The HCP server 416 hosts the HCP server process 432 that communicates with the analysis engine 130 and the applications on the mobile device 110.

For example, the HCP server process 432 or a similar process on the data server 412 may provide compliance analysis based on the use of the RPT 4000 in accordance with compliance rules that specify the required RPT usage over a compliance period, such as thirty (30) days, in terms of a minimum duration of device usage per session, such as four hours, for some minimum number of days, e.g. 21, within the compliance period. A session is deemed compliant if its duration exceeds the minimum duration. The usage data post-processing may determine whether the most recent session is a compliant session by comparing the usage duration with the minimum duration from the compliance rule. The result of such post-processing is compliance data, such as a Boolean compliance variable, that forms part of the usage data. A further example of multi-session usage data is a count of compliant sessions since the start of RPT therapy. The summary data post-processing may determine whether the most recent time period is a compliant session by comparing the usage time with the minimum duration from the compliance rule. Such compliance data may be used by a health care provider to tailor therapy that may include the inhaler and other mechanisms. Other actors such as payors may use the compliance data to determine whether reimbursement may be made to a patient.

The data may also be provided to the support server 418 that may run a process to insure that supply chains are alerted to provide replacement components or parts forthe RPT 4000. The support server 418 may also issue alerts to service providers to provide maintenance or repair of the RPT 4000.

As may be appreciated, data in the data server 412, EMR server 414 and HCP server 416, and support server 418 is generally confidential data in relation to the patients in the system 400. Typically, patients must provide permission to send the confidential data to another party. Such permissions may be required to transfer data between servers 412, 414, 416, and 418 if such servers are operated by different entities.

The system 400 incorporates the health hub platform system 100 in FIG. 1A. The system 400 may include AR/VR (augmented reality/virtual reality) health care professional consultation based on detected patient issues from the health analysis engine 130. Such consultation may be provided by patient-accessible devices such as the mobile device 110 or other media devices such as a smart TV. For a scheduled check-in, the system 400 can provide health care providers such as physicians recent and trend data as well as a prediction of future changes (getting better, getting worse, stable) of the patient 1000. For unscheduled check-ins, the system 400 can outline why the check-in is occurring (e.g., whether the system, the patient, or the physician/clinician/caregiver has triggered the check). Such information may be available from a network-enabled device that is accessible to the physician/clinician/caregiver such as the work station 170 in FIG. 1.

For real time or near real time intervention, appropriate processing and communications may be used. Technically, if New Radio 5Gisused, the deployment could include equipment placed into carrier's network (edge cloud processing) and /or "slicing" fast path (logical separation) in a carrier's network to insure critical care alerts are delivered to a physician/health care entity/first responder, etc. The data connection between devices in FIG. 4 can use slicing as part of assurance around connectivity, and information security.

In terms of service delivery, this could include automatically creating a health care facility appointment, and booking transport (e.g., a taxi, an autonomous vehicle, an ambulance) to a health care facility. Such services may be provided by the process 432 of the HCP server 416, either alone or in conjunction with processes executed by the support server 418.

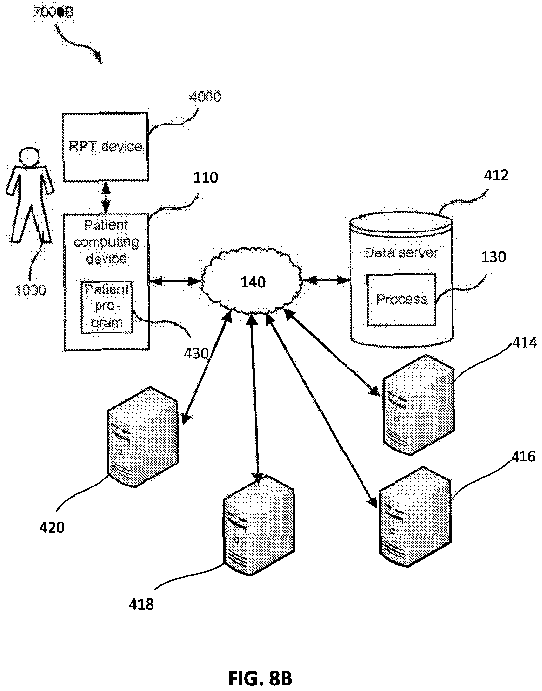

FIG. 8B contains a block diagram illustrating an alternative implementation 7000B of an RPT system according to the present technology. In the alternative implementation 7000B, the RPT device 4000 communicates with the patient computing device 110 via a local (wired or wireless) communications protocol such as a local network protocol (e.g., Bluetooth). In the alternative implementation 7000B, the local network may be identified with the local external communication network 4284 of FIG. 4C, and the patient computing device 110 may be identified with the local external device 4288 of FIG. 4C. In the alternative implementation 7000B, the patient computing device 110, via the patient program 430, is configured to intermediate between the patient 1000 and the data server 412, over the wide area network 140, and also between the RPT device 4000 and the data server 412 over the wide area network 140.

In what follows, statements about the RPT system 400 may be understood to apply equally to the alternative implementation 7000B, except where explicitly stated otherwise. For example, other servers such as the EMR server 414, the HCP server 416, the supply and support server 418, and the payor data server 420 may communicate with each other and the patient computing device 110 through the network 140.

In this example, the RPT device 4000 is configured to store in the memory 4260 therapy data from each RPT session delivered to the patient 1000. Therapy data for an RPT session comprises the settings of the RPT device 4000 and therapy variable data representing one or more variables of the respiratory pressure therapy throughout the RPT session.

The RPT device 4000 is configured to transmit the therapy data to the data server 7010. As explained above, the transmission of the data is modulated based on different cases. In normal operation, low resolution data alone is transmitted. High resolution data may be transmitted in different cases, as will be explained below. The data server 412 may receive the therapy data from the RPT device 4000 according to a "pull" model whereby the RPT device 4000 transmits the therapy data in response to a query from the data server 412. Alternatively, the data server 412 may receive the therapy data according to a "push" model whereby the RPT device 4000 transmits the therapy data to the data server 412 as soon as convenient after an RPT session.

Therapy data received from the RPT device 4000 is stored and indexed by the data server 412 so as to be uniquely associated with the RPT device 4000 and therefore distinguishable from therapy data from any other RPT device(s) participating in the RPT system 400.

In this example, the data server 412 is configured to calculate different types of analytical data that may be of use to a clinician. For example, usage data for each RPT session may be determined from the therapy data received from the RPT device 4000. Usage data variables for a session comprise summary statistics derived by conventional scoring means from the therapy variable data that forms part of the therapy data. Usage data may comprise one or more of the following usage variables: a) Usage time, i.e. total duration of the RPT session; b) Apnea-hypopnea index (AHI) for the session; c) Average leak flow rate for the session; d) Average mask pressure for the session; e) Number of "sub-sessions" within the RPT session, i.e. number of intervals of RPT therapy between "mask-on" and "mask-off" events; and f) Other statistical summaries of the therapy variables, e.g. 95th percentile pressure, median pressure, histogram of pressure values. Usage variables may comprise multi-session statistics, such as mean, median, and variance of AHI since the start of RPT therapy.

Other servers may be coupled to the network 140 and obtain data based on the "push" or "pull" model described above. For example, the data server 420 operated by a payor may receive data for different purposes such as determining compliance or predicting compliance for purposes of determining payment for therapy of the patient 1000. A machine learning server that executes the machine learning engine 180 in FIG. 1A may also receive data for purposes of learning or refining baselines for exceptional cases that require high resolution data as will be explained below. Alternatively, the machine learning server may learn optimal responses when exceptional cases are detected or the correct predictive data to be included in high resolution data in response to certain exceptional cases.

In an alternative implementation, the RPT device 4000 calculates the usage variables from the therapy data stored by the RPT device 4000 at the end of each session. The RPT device 4000 then transmits the usage variables to the data server 7010 according to the "push" or "pull" model described above.

In a further implementation, the memory 4260 in which the RPT device 4000 stores the therapy/usage data for each RPT session is in removable form, such as an SD memory card. The removable memory 4260 may be removed from the RPT device 4000 and inserted into a card reader in communication with the data server 412. The therapy/usage data is then copied from the removable memory 4260 to the memory of the data server 412.

In still a further implementation, suitable for the alternative implementation 7000B of the RPT system, the RPT device 4000 is configured to transmit the therapy/usage data to the patient computing device 110 via a wireless communications protocol such as Bluetooth as described above. The patient computing device 110 then transmits the therapy/usage data to the data server 412. The data server 412 may receive the therapy/usage data from the patient computing device 110 according to a "pull" model whereby the patient computing device 110 transmits the therapy/usage data in response to a query from the data server 412. Alternatively, the data server 412 may receive the therapy/usage data according to a "push" model whereby the patient computing device 110 transmits the therapy/usage data to the data server 412 as soon as it is available after an RPT session.

The data server412 may also be configured to receive data from the patient computing device 110. Such may include data entered by the patient 1000 to the patient program 430, or therapy/usage data in the alternative implementation 7000B described above.

The data server 412 is also configured to transmit electronic messages to the patient computing device 110. The messages may be in the form of emails, SMS messages, automated voice messages, or notifications within the patient program 430.

The RPT device 4000 may be configured such that its therapy mode, or settings for a particular therapy mode, may be altered on receipt of a corresponding command via its wide area or local area network connection. In such an implementation, the data server 412 may also be configured to send such commands directly to the RPT device 4000 (in the implementation 400) or indirectly to the RPT device 4000, relayed via the patient computing device 110 (in the implementation 7000B).

The data server 412 may host a process run by the analysis engine 130, described in detail below, that is configured to increase or sustain the patient's motivation to continue with therapy. In broad terms, the process analyses data from the RPT device 4000 and/or the patient computing device 110 to compute a therapy quality indicator that is indicative of the quality of the most recent therapy session. The process then communicates the therapy quality indicator to the patient 1000, for example via the patient program 430 running on the patient computing device 110. The need to increase motivation may be detected by analysing low resolution data and high resolution data may be obtained to optimize the methods to increase or sustain the motivation of the patient.

The patient 1000 perceives the therapy quality indicator as a concise indicator of how their therapy is going. The patient 1000 is thereby motivated to persevere with their therapy. It is known that tracking and measuring performance can be a strong motivator for a person to achieve their goals, and the therapy quality indicator serves as such a performance measure in the context of respiratory pressure therapy.

Generally speaking, the level of data thatis regularly/routinely transmitted to the data server 412 is summarized information that is sufficient to support the patient, however in some cases higher data resolution is required either to drill into an issue further for an actor such as a health care provider or payer, or to provide data back to the manufacturer about the performance of the machine. Thus, an advantage to differentiating between low resolution data and high resolution data transmission is preventing data overload that is typically unnecessary, except in special situations. The ability to shift the volume of transmitted data allows the use of more efficienttransmission components that do notrequirethe highest level of bandwidth for the system.

The cases which may trigger the higher data resolution may be predetermined by the manufacturer, a payer or a health care provider. Such cases may also be determined by using a rules engine executed by the controller 4230. The rules engine may be determined by machine learning routines executed by the machine learning engine 180 to identify when these cases have occurred and request that the relevant high resolution data is sent by the controller 4230 to the remote external device 4286 such as the cloud.

The rules engine may perform simple analysis to identify negative trends, alerting the system when thresholds have been breached. Machine learning would be used to understand what a "normal" condition may be for a patient (creating baselines) and help identify abnormalities in the operation of the RPT device 4000. In these cases, specific high resolution data could be sent for a specified period of time such as a few days or until feedback data from the controller 4230 allows identification that the problem is resolved. Examples of cases and corresponding high resolution data could include a trend of high leak that is determined from the low resolution data. Such a trend exceeding a baseline, may results in sending high resolution leak data. Another case may be an increasing respiratory rate thatis determined from the low resolution data. On detection of the increasing respiratory rate, the controller 4230 may send high resolution respiratory rate, respiratory flow, pressure and flow limitation data to the external device 4286.

Another case may be continual desaturations (e.g., low SpO2 values) that may be detected from the low resolution data. On detection of the increasing respiratory rate, the controller 4230 sends high resolution SpO2, pressure and flow limitation data to the external device 4286. Another case may be a combination of increased cough, sputum and respiratory rate that may be detected from the low resolution data. On detection of the increased cough, sputum and respiratory rate, the controller 4230 sends high resolution respiratory rate, SpO2, and pressure data to the external device 4286.

In this example, low resolution data that may be sampled by the controller 4230 and selected for low frequency transmissionto the external device 4286 may include SpO2, Pulse Rate, Respiratory Events, treatment pressure (IPAP), base pressure (EPAP), Leak, Minute Ventilation, Tidal Volume, Respiratory Rate, Spontaneous Trigger percentage, Spontaneous Cycle percentage, I:E Ratio, Inhaler events, Sleep State detection, Mouth leak, Last known exacerbation, Dsypnea, Activity Level, Sputum production/color, Cough, Delivered oxygen level, Breath morphology, Temperature, Humidity, Air Quality, Rescue medication use, Body temperature, Peak expiratory Flow rate, FEV1, or Vital capacity. As explained above, any or all of the above data types may be set at a low rate of sampling or transmission such as once per minute. The high resolution data may be the same data with a higher rate of sampling such as greater than once per minute.

High resolution data may constitute additional low resolution data as explained above. High resolution data may also include types such as Respiratory Flow, Pressure, Trigger / Cycle events, Mask pressure, IPAP, EPAP, Leak, Respiratory Rate, Tidal Volume, Spontaneous Trigger percentage, Spontaneous Cycle percentage, I:E Ratio, Snore, Flow Limitation, Pulse Rate, SpO2, Time in inspiration, Average inspiration time for last 5 breaths, Average expiration time for last 5 breaths, Cough, Breath morphology, Activity Level, Peak expiratory flow rate, FEV1, Vital capacity, and Sleep State detection.

Low or high resolution data may also be obtained by other therapy or sensor devices that may be interfaced with the controller 4230 or the patient computer 110. This data may be combined with or included in transmissions from the device 4000.

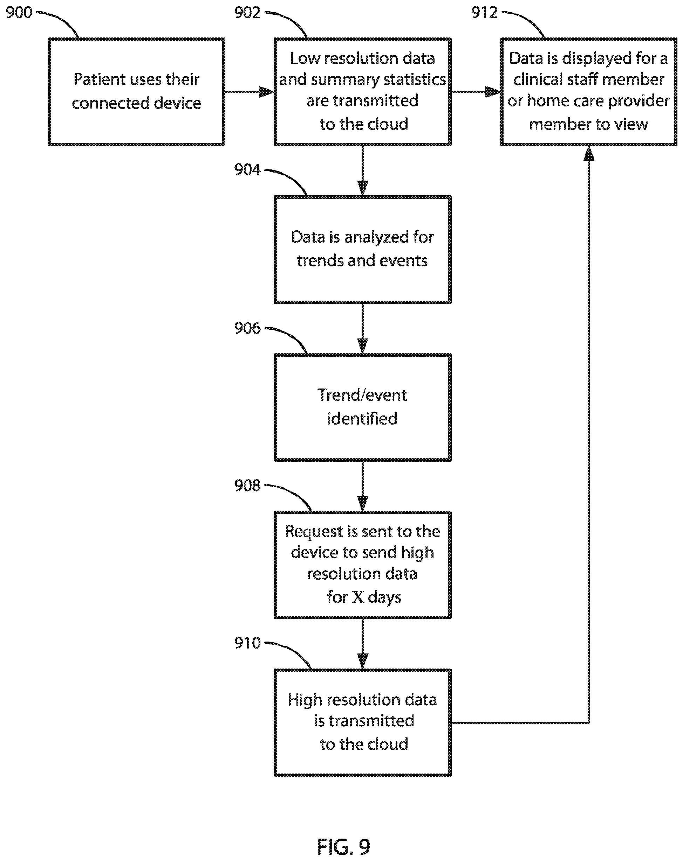

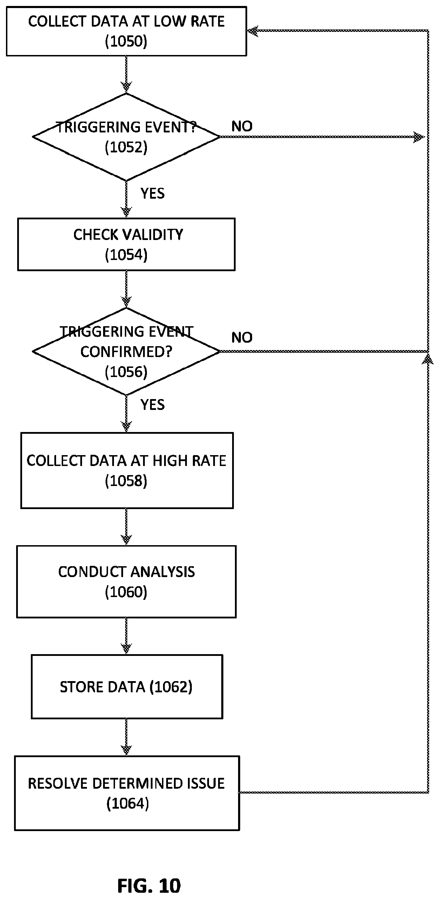

FIG. 9 shows a flow diagram of the routine executed by the controller 4230 in FIG. 4C for regulating transmission of data. FIG. 10 shows a flow diagram for the collection and analysis routine to collect data for determining health conditions for the patient 1000 in FIG. 1A. The flow diagram in FIG. 9 is representative of example machine readable instructions for collecting and regulating the transmission of data. The flow diagram in FIG. 10 is representative of example machine readable instructions for collection and analysis of data based on triggering events for determining health conditions. In this example, the machine readable instructions comprise an algorithm for execution by: (a) a processor; (b) a controller; and/or (c) one or more other suitable processing device(s). The algorithm may be embodiedin software stored on tangible media such as flash memory, CD-ROM, floppy disk, hard drive, digital video (versatile) disk (DVD), or other memory devices. However, persons of ordinary skill in the art will readily appreciate that the entire algorithm and/or parts thereof can alternatively be executed by a device otherthan a processor and/or embodied in firmware or dedicated hardware in a well-known manner (e.g., it may be implemented by an application specific integrated circuit [ASIC], a programmable logic device [PLD], a field programmable logic device [FPLD], a field programmable gate array [FPGA], discrete logic, etc.). For example, any or all of the components of the interfaces can be implemented by software, hardware, and/or firmware. Also, some or all of the machine readable instructions represented by the flowcharts may be implemented manually. Further, although the example algorithm is described with reference to the flowcharts illustrated in FIGs. 9 and 10, persons of ordinary skill in the art will readily appreciate that many other methods of implementing the example machine readable instructions may alternatively be used. For example, the order of execution of the blocks may be changed, and/or some of the blocks described may be changed, eliminated, or combined.

In relation to FIG. 9, the patient uses the connected device such as the RPT device 4000 in FIG. 8A (900). The device 4000 collects various data that may be either low resolution or high resolution data when in use. As explained above, the collected data is stored in local memory 4260 on the device 4000. The low resolution data and summary statistics compiled by the device are transmitted to the external system such as the cloud 140 in FIG. 8A (902). The low resolution data is analysed for trends and events using external applications that may be executed by servers on the cloud such as the server 412 or the server 420 (904).

As explained above, a trend or event may be identified by applications that signal an exceptional event relating to the patient (906). A request is then sent to the device 4000 to send high resolution data (908). In this example, the high resolution data is defined based on the detected trend or event. Further, in this example, the high resolution data is sent for a predetermined amount of time such as several days. The collected high resolution data is transmitted to the external device such as the server 412 (910). The cloud 7090 allows access to the high resolution data from an authorized actor such as a health care professional or a payer through a web-based interface (912).

FIG. 10 shows a flow diagram for the collection and analysis routine performed by the system 100 specific to determination of health conditions. The system first monitors data collected at a low resolution mode from the RPT device 4000 (1050). In this example, the data analysis engine 130 collects the low resolution data and analyzes the data for a triggering event, such as a change in the condition of the patient. As explained above, the triggering events may be predetermined rules or machine-learning-based models or algorithms. The analysis engine 130 determines whether a triggering event has occurred based on the collected data from the low resolution mode (1052). If there is no triggering event detected, the routine continues to collect data at a low resolution (1050).

If a triggering event has been detected (1052), the health analysis engine 130 confirms the triggering event (1054). The confirmation of the triggering event may include collecting additional types of data or following a set rule to cross-check the occurrence of the triggering event. Such collection may be made on an application on the mobile device 110. The analysis engine 130 then determines whether the triggering event is confirmed (1056). If the triggering event is not confirmed, the routine continues to collect data at a low resolution (1052). If the triggering event is confirmed, the analysis engine 130 controls the RPT device 4000 and other relevant sources to increase the collection of data to a high resolution (1058). As explained above, additional data may be from the patient health monitor 120, other sensors, and other databases. The data analysis engine 130 then analyzes the collected data to determine a health condition

(1060). The health condition analysis may include determining the severity of the triggering condition. The collected analysis is then stored (1062) for further analysis and other purposes such as building on a training set for the machine-learning engine 180. The health analysis engine 130 then determines and implements a resolution for the detected triggering event (1064). For example, this may involve notifications to the patient in less severe triggering events or notifications to a health care professional in more severe triggering events. Other examples may be adjustment of the settings of the RPT device 4000 and automatic adjustment of treatment or medication. Once the data analysis engine 130 determines the triggering event has been resolved, it returns to the collection of data at a low resolution (1050).

In terms of service delivery, this could include automatically creating a health care facility appointment, and booking transport (e.g., a taxi, an autonomous vehicle, an ambulance) to a health care facility. Such services may be provided by the process 432 of the HCP server 416, either alone or in conjunction with processes executed by the support server 418.

There are numerous benefits of the system that collects data from respiratory therapy devices for analyzing heath conditions. Benefits to the example patient 1000 includes increased quality of life and reduced hospitalizations, maximizing long term adherence (LTA), the ability to interface with an application executed by the mobile device 110, and the ability to contact emergency services if unusual data detected. Quality of life may include aspects of physical, psychological, and social functioning, and may include a feeling (or absence of) overall life satisfaction.

Benefits for the health care provider (such as an integrated care provider) include understanding the evolution of co-morbidities thus keeping patients out of the hospital, personalization of the therapy experience, attract direct to patient/customer out-of-pocket purchases.

### 4.11 GLOSSARY

For the purposes of the present technology disclosure, in certain forms of the present technology, one or more of the following definitions may apply. In other forms of the present technology, alternative definitions may apply.

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be differentto the humidity outside the room where a patient is sleeping.

In another example, ambient pressure may be the pressure immediately surrounding or external to the body.

In certain forms, ambient(e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy*: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Patient*: A person, whether or not they are suffering from a respiratory condition.

*Pressure*: Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT)*: The application of a supply of air to an entrance to the airways at a treatment pressure that is typically positive with respect to atmosphere.

*Ventilator*: A mechanical device that provides pressure support to a patient to perform some or all of the work of breathing.

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a," "an," and "the" include their plural equivalents, unless the context clearly dictates otherwise.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

## Claims

1. A patient treatment device (4000), comprising:
a memory (4260);
a first sensor (4272, 4274, 4276, 4278, 4279), wherein the first sensor is configured to collect a first type of data from a patient, wherein the collected first type of data is stored in the memory;
a controller (4230) coupled to the first sensor, the controller operable to:
control the memory to store low resolution data of the first type of data collected at a lower sampling rate;
determine the occurrence of a triggering event that includes provision of new medication or treatment to the patient; and
control the memory to store higher resolution data of the first type of data collected at a higher sampling rate on occurrence of the triggering event; and
a transmitter (4280), wherein the transmitter (4280) is controlled by the controller (4230) to send the low resolution data or the high resolution data to the external system (110, 412).

2. The patient treatment device according to claim 1, wherein the patient treatment device is a respiratory pressure therapy (RPT) device, optionally wherein the first sensor is one of a flow rate sensor or a pressure sensor.

3. The patient treatment device according to either claim 1 or claim 2, further comprising a second sensor (4272, 4274, 4276, 4278, 4279), wherein the second sensor is configured to collect a second type of data from the patient; and wherein the high resolution data includes the second type of data.

4. The patient treatment device according to any one of claims 1 to 3, where the triggering event is a request received from the external device based on a specific purpose.

5. The patient treatment device according to any one of claims 1 to 4, wherein the triggering event is a request to build a health record of a patient, and wherein the higher resolution collection occurs over a predetermined period of time associated with the health record.

6. The patient treatment device according to any one of claims 1 to 5, wherein the triggering event is a request to analyze a health condition over a predetermined period of time.

7. The patient treatment device according to any one of claims 1 to 6, wherein the external device includes a rules engine performing analysis on the low resolution data to identify a negative trend relating to health of the patient, wherein the triggering event is when the negative trend exceeds a predetermined threshold.

8. The patient treatment device according to claim 7, wherein the controller is operable to sending high resolution data until the rules engine determines the negative trend has been resolved.

9. The patient treatment device according to claim 2, wherein the RPT device is configured to store therapy data from an RPT session delivered to a patient in the memory, wherein the therapy data for an RPT session including settings of the RPT device and therapy variable data representing one or more variables of the RPT throughout the RPT session, or
wherein the external system includes a data server, and wherein the RPT device transmits the data according to a pull model whereby the RPT device transmits the data in response to a query from the data server, or wherein the RPT device transmits the data according to a push model whereby the RPT device transmits the data to the data server after an RPT session,
optionally wherein the external system includes comprising multiple patient treatment devices, wherein the external system comprises a data server, the therapy data received from each of the patient treatment devices being stored and indexed by the data server so as to be uniquely associated with the respective patient treatment device.

10. The patient treatment device according to any one of claims 1 to 9, wherein the external system is a personal computer, a mobile phone, a tablet, or a remote control; and/or wherein the external system includes a health analysis engine operable to determine a severity of the detected event and adjust a responsive action to the patient based on the determined severity,
optionally wherein the health analysis engine is operative to request subjective input data entered by the patient to verify the detection of the event.

11. The patient treatment device according to any one of claims 1 to 10, wherein the controller is operable to adjust operation of the patient treatment device in response to the trigger event.

12. The patient treatment device according to any one of claims 1 to 11, wherein the low resolution data comprises any one or any combination of data types of SpO2, Pulse Rate, Respiratory Events, treatment pressure (IPAP), base pressure (EPAP), Leak, Minute Ventilation, Tidal Volume, Respiratory Rate, Spontaneous Trigger percentage, Spontaneous Cycle percentage, I:E Ratio, Inhaler events, Sleep State detection, Mouth leak, Last known exacerbation, Dsypnea, Activity Level, Sputum production/color, Cough, Delivered oxygen level, Breath morphology, Temperature, Humidity, Air Quality, Rescue medication use, Body temperature, Peak expiratory Flow rate, FEV1, and Vital capacity, and/or
wherein the high resolution data comprises any one or any combination of data types of Respiratory Flow, Pressure, Trigger/Cycle events, Mask pressure, IPAP, EPAP, Leak, Respiratory Rate, Tidal Volume, Spontaneous Trigger percentage, Spontaneous Cycle percentage, I:E Ratio, Snore, Flow Limitation, Pulse Rate, SpO2, Time in inspiration, Average inspiration time for last 5 breaths, Average expiration time for last 5 breaths, Cough, Breath morphology, Activity Level, Peak expiratory flow rate, FEV1, Vital capacity, and Sleep State detection.

13. The patient treatment device according to any one of claims 1 to 12, wherein the controller is operable to perform a verification of a real triggering event, the verification including a diagnostic on the device (4000) to determine whether an error occurred in the operation of the device, and wherein the high resolution data is transmitted based on the verification of a real triggering event.

14. A method to modulate the transmission of patient data from a patient treatment device (4000) to an external system (110, 412), the patient treatment device including a first sensor (4272, 4274, 4276, 4278, 4279) coupled to a patient, a memory (4260), and a transmitter (4280), the method comprising:
collecting a first type from the first sensor (4272, 4274, 4276, 4278, 4279);
storing low resolution data collected from the first sensor at a lower sampling rate;
determining the occurrence of a triggering event that includes a provision of new medication or treatment to the patient;
storing high resolution data collected from the first sensor at a higher sampling rate on occurrence of the triggering event; and
transmitting either the low resolution data or the high resolution data to the external device via the transmitter.

## Patentansprüche

1. Vorrichtung (4000) zur Behandlung von Patienten, umfassend:
einen Speicher (4260);
einen ersten Sensor (4272, 4274, 4276, 4278, 4279), wobei der erste Sensor dazu ausgestaltet ist, einen ersten Datentyp von einem Patienten zu sammeln, wobei der gesammelte erste Datentyp in dem Speicher gespeichert wird,
ein Steuergerät (4230), das mit dem ersten Sensor gekoppelt ist, wobei das Steuergerät betätigbar ist, um:
den Speicher zu steuern, Daten mit niedriger Auflösung des ersten Datentyps zu speichern, die mit einer geringeren Abtastrate gesammelt werden;
das Vorkommen eines Auslösungsereignisses zu bestimmen, das Bereitstellen einer neuen Medikation oder Behandlung für den Patienten beinhaltet; und
den Speicher zu steuern, Daten mit höherer Auflösung des ersten Datentyps, die mit einer höheren Abtastrate gesammelt werden, beim Vorkommen des Auslösungsereignisses zu speichern; und
einen Sender (4280), wobei der Sender (4280) durch das Steuergerät (4230) gesteuert wird, um entweder die Daten mit niedriger Auflösung oder die Daten mit hoher Auflösung an das externe System (110, 412) zu übertragen.

2. Vorrichtung zur Behandlung von Patienten nach Anspruch 1, wobei die Vorrichtung zur Behandlung von Patienten eine Vorrichtung zur Beatmungsdrucktherapie (RPT) ist, wobei optional der erste Sensor einer von einem Flussratensensor oder einem Druckmessfühler ist.

3. Vorrichtung zur Behandlung von Patienten nach Anspruch 1 oder Anspruch 2, ferner umfassend einen zweiten Sensor (4272, 4274, 4276, 4278, 4279), wobei der zweite Sensor dazu ausgestaltet ist, einen zweiten Datentyp von einem Patienten zu sammeln; und wobei die Daten mit hoher Auflösung den zweiten Datentyp beinhalten.

4. Vorrichtung zur Behandlung von Patienten nach einem der Ansprüche 1 bis 3, wobei das Auslösungsereignis eine Anfrage ist, die von der externen Vorrichtung auf Grundlage eines spezifischen Zwecks empfangen wird.

5. Vorrichtung zur Behandlung von Patienten nach einem der Ansprüche 1 bis 4, wobei das Auslösungsereignis eine Anfrage ist, eine Patientenakte eines Patienten zu erstellen, und wobei die Sammlung höherer Auflösung über einen vorbestimmten Zeitraum erfolgt, der mit der Patientenakte verknüpft ist.

6. Vorrichtung zur Behandlung von Patienten nach einem der Ansprüche 1 bis 5, wobei das Auslösungsereignis eine Anfrage ist, einen Gesundheitszustand über einen vorbestimmten Zeitraum zu analysieren.

7. Vorrichtung zur Behandlung von Patienten nach einem der Ansprüche 1 bis 6, wobei die externe Vorrichtung eine Regel-Engine beinhaltet, die eine Analyse der Daten mit niederer Auflösung durchführt, um einen negativen Trend in Bezug auf eine Gesundheit des Patienten zu identifizieren, wobei das Auslösungsereignis eintritt, wenn der negative Trend einen vorbestimmten Schwellenwert übersteigt.

8. Vorrichtung zur Behandlung von Patienten nach Anspruch 7, wobei das Steuergerät betätigbar ist, Daten mit hoher Auflösung zu übertragen, bis die Regel-Engine bestimmt, dass der negative Trend aufgelöst wurde.

9. Vorrichtung zur Behandlung von Patienten nach Anspruch 2, wobei die RPT-Vorrichtung dazu ausgestaltet ist, Therapiedaten von einer RPT-Sitzung, die einem Patienten verabreicht wurde, in dem Speicher zu speichern, wobei die Therapiedaten für eine RPT-Sitzung Einstellungen der RPT-Vorrichtung und Therapie-Variablendaten beinhalten, die eine oder mehrere Variablen der RPT über den Verlauf der RPT-Sitzung darstellen, oder
wobei das externe System einen Datenserver umfasst, und wobei die RPT-Vorrichtung die Daten in Übereinstimmung mit einem Pull-Modell überträgt, wodurch die RPT-Vorrichtung die Daten in Antwort auf eine Anfrage von dem Datenserver überträgt, oder wobei die RPT-Vorrichtung die Daten in Übereinstimmung mit einem Push-Modell überträgt, wodurch die RPT-Vorrichtung die Daten nach einer RPT-Sitzung an den Datenserver überträgt,
wobei das externe System optional beinhaltet, dass mehrere Vorrichtungen zur Behandlung von Patienten umfasst sind, wobei das externe System einen Datenserver umfasst, wobei die Therapiedaten, die von jeder der Vorrichtungen zur Behandlung von Patienten kommend empfangen werden, von dem Datenserver gespeichert und indexiert werden, so dass sie eineindeutig der jeweiligen Vorrichtung zur Behandlung von Patienten zugeordnet sind.

10. Vorrichtung zur Behandlung von Patienten nach einem der Ansprüche 1 bis 9, wobei das externe System ein Personalcomputer, ein Mobiltelefon, ein Tablet-Computer oder eine Fernsteuerung ist; und/oder wobei das externe System eine Gesundheits-Analyse-Engine beinhaltet, die betätigbar ist, um einen Schweregrad des erkannten Ereignisses zu bestimmen und eine entsprechende Aktion an dem Patienten auf Grundlage des bestimmten Schweregrads einzustellen,
wobei optional die Gesundheits-Analyse-Engine betätigbar ist, um subjektive Eingabedaten, die von dem Patienten eingegeben werden, anzufordern, um die Erkennung des Ereignisses zu verifizieren.

11. Vorrichtung zur Behandlung von Patienten nach einem der Ansprüche 1 bis 10, wobei das Steuergerät betätigbar ist, einen Betrieb der Vorrichtung zur Behandlung von Patienten in Reaktion auf das Auslösungsereignis anzupassen.

12. Vorrichtung zur Behandlung von Patienten nach einem der Ansprüche 1 bis 11, wobei die Daten mit niedriger Auflösung einen beliebigen der Datentypen SpO2, Pulsfrequenz, respiratorische Ereignisse, Behandlungsdruck (IPAP), Basisdruck (EPAP), Leckage, Minutenbeatmung, Tidalvolumen, Atemfrequenz, Spontantrigger-Prozentsatz, Spontanzyklus-Prozentsatz, l:E-Verhältnis, Einatmungsereignisse, Erkennung des Schlafzustands, Mundleckage, letzte bekannte Exazerbation, Dyspnoe, Aktivitätsniveau, Sputumproduktion/-farbe, Husten, abgegebener Sauerstoffgehalt, Atemmorphologie, Temperatur, Luftfeuchtigkeit, Luftqualität, Verwendung von Notfallmedikamenten, Körpertemperatur, exspiratorische Spitzenflussrate, FEV1 und Vitalkapazität oder eine beliebige Kombination umfassen; und/oder
wobei die Daten mit hoher Auflösung einen beliebigen der Datentypen Atemfluss, Druck, Trigger/Zyklus-Ereignisse, Maskendruck, IPAP, EPAP, Leckage, Atemfrequenz, Tidalvolumen, Spontantrigger-Prozentsatz, Spontanzyklus-Prozentsatz, l:E-Verhältnis, Schnarchen, Flussbegrenzung, Pulsfrequenz, SpO2, Inspirationszeit, durchschnittliche Inspirationszeit für die letzten 5 Atemzüge, durchschnittliche Exspirationszeit für die letzten 5 Atemzüge, Husten, Atemmorphologie, Aktivitätsniveau, Spitzen-Exspirationsflussrate, FEV1, Vitalkapazität und Schlafzustandserkennung oder eine beliebige Kombination davon umfassen.

13. Vorrichtung zur Behandlung von Patienten nach einem der Ansprüche 1 bis 12, wobei das Steuergerät betätigbar ist, um eine Verifizierung eines realen Auslösungsereignisses durchzuführen, wobei die Verifizierung ein Diagnosemittel an der Vorrichtung (4000) beinhaltet, um zu bestimmen, ob ein Fehler in dem Betrieb der Vorrichtung aufgetreten ist, und wobei die Daten mit hoher Auflösung auf der Grundlage der Verifizierung eines realen Auslösungsereignisses übertragen werden.

14. Verfahren zum Modulieren der Übertragung von Patientendaten von einer Vorrichtung zur Behandlung von Patienten (4000) an ein externes System (110, 412), wobei die Vorrichtung zur Behandlung von Patienten einen ersten Sensor (4272, 4274, 4276, 4278, 4279), der mit einem Patienten gekoppelt ist, einen Speicher (4260), und einen Sender (4280) beinhaltet, wobei das Verfahren umfasst:
Sammeln eines ersten Typs von dem ersten Sensor (4272, 4274, 4276, 4278, 4279);
Speichern von Daten mit niedriger Auflösung, die von dem ersten Sensor mit einer geringeren Abtastrate gesammelt werden;
Bestimmen des Vorkommens eines Auslösungsereignisses, das Bereitstellen einer neuen Medikation oder Behandlung für den Patienten beinhaltet;
Steuern von Daten mit hoher Auflösung, die von dem ersten Sensor mit einer höheren Abtastrate gesammelt werden, beim Vorkommen des Auslösungsereignisses; und
Übertragen entweder der Daten mit niedriger Auflösung oder der Daten mit hoher Auflösung an die externe Vorrichtung über den Sender.

## Revendications

1. Dispositif de traitement de patient (4000), comprenant :
une mémoire (4260) ;
un premier capteur (4272, 4274, 4276, 4278, 4279), dans lequel le premier capteur est configuré pour collecter un premier type de données d'un patient, dans lequel le premier type de données collecté est stocké dans la mémoire ;
un dispositif de commande (4230) couplé au premier capteur, le dispositif de commande étant apte à :
commander la mémoire pour stocker des données à basse résolution du premier type de données collectées à un taux d'échantillonnage inférieur ;
déterminer la survenue d'un événement déclencheur qui inclut la fourniture d'un nouveau médicament ou traitement au patient ; et
commander la mémoire pour stocker des données à plus haute résolution du premier type de données collectées à un taux d'échantillonnage supérieur lors de la survenue de l'événement déclencheur ; et
un émetteur (4280), dans lequel l'émetteur (4280) est commandé par le dispositif de commande (4230) pour envoyer les données à faible résolution ou les données à haute résolution au système externe (110, 412).

2. Dispositif de traitement de patient selon la revendication 1, dans lequel le dispositif de traitement de patient est un dispositif de thérapie respiratoire par pression (RPT), éventuellement dans lequel le premier capteur est l'un d'un capteur de débit ou d'un capteur de pression.

3. Dispositif de traitement de patient selon la revendication 1 ou la revendication 2, comprenant en outre un second capteur (4272, 4274, 4276, 4278, 4279), dans lequel le second capteur est configuré pour collecter un second type de données du patient ; et dans lequel les données à haute résolution incluent le second type de données.

4. Dispositif de traitement de patient selon l'une quelconque des revendications 1 à 3, dans lequel l'événement déclencheur est une demande reçue du dispositif externe, basée sur un objectif spécifique.

5. Dispositif de traitement de patient selon l'une quelconque des revendications 1 à 4, dans lequel l'événement déclencheur est une demande pour établir un dossier de santé d'un patient, et dans lequel la collecte à plus haute résolution se produit sur un laps de temps prédéterminé associé au dossier de santé.

6. Dispositif de traitement de patient selon l'une quelconque des revendications 1 à 5, dans lequel l'événement déclencheur est une demande d'analyse d'un état de santé sur un laps de temps prédéterminé.

7. Dispositif de traitement de patient selon l'une quelconque des revendications 1 à 6, dans lequel le dispositif externe inclut la réalisation par un moteur de règles d'une analyse sur les données à basse résolution pour identifier une tendance négative relative à la santé du patient, dans lequel l'événement déclencheur se produit quand la tendance négative dépasse un seuil prédéterminé.

8. Dispositif de traitement de patient selon la revendication 7, dans lequel le dispositif de commande est apte à envoyer des données haute résolution jusqu'à ce que le moteur de règles détermine que la tendance négative a été résolue.

9. Dispositif de traitement de patient selon la revendication 2, dans lequel le dispositif RPT est configuré pour stocker des données de thérapie provenant d'une session RPT délivrée à un patient dans la mémoire, dans lequel les données de thérapie pour une session RPT incluent des paramètres du dispositif RPT et des données de variables de thérapie représentant une ou plusieurs variables de la RPT tout au long de la session RPT, ou
dans lequel le système externe inclut un serveur de données, et dans lequel le dispositif RPT transmet les données selon un modèle pull par lequel le dispositif RPT transmet les données en réponse à une requête du serveur de données, ou dans lequel le dispositif RPT transmet les données selon un modèle push par lequel le dispositif RPT transmet les données au serveur de données après une session RPT,
éventuellement dans lequel le système externe inclut de multiples dispositifs de traitement de patient, dans lequel le système externe comprend un serveur de données, les données de thérapie reçues de chacun des dispositifs de traitement de patient étant stockées et indexées par le serveur de données de manière à être associées de manière unique au dispositif de traitement de patient respectif.

10. Dispositif de traitement de patient selon l'une quelconque des revendications 1 à 9, dans lequel le système externe est un ordinateur personnel, un téléphone mobile, une tablette ou une télécommande ; et/ou dans lequel le système externe inclut un moteur d'analyse de la santé pouvant fonctionner pour déterminer une gravité de l'événement détecté et adapter une action réactive au patient en fonction de la gravité déterminée,
éventuellement dans lequel le moteur d'analyse de la santé fonctionne pour demander des données d'entrée subjectives entrées par le patient pour vérifier la détection de l'événement.

11. Dispositif de traitement de patient selon l'une quelconque des revendications 1 à 10, dans lequel le dispositif de commande peut fonctionner pour ajuster le fonctionnement du dispositif de traitement de patient en réponse à l'événement déclencheur.

12. Dispositif de traitement de patient selon l'une quelconque des revendications 1 à 11, dans lequel les données à faible résolution comprennent l'un quelconque ou une combinaison quelconque des types de données parmi SpO2, Fréquence du Pouls, Événements Respiratoires, pression de traitement (IPAP), pression de base (EPAP), Fuite, Ventilation Minute, Volume Courant, Fréquence Respiratoire, Pourcentage de Déclenchement spontané, Pourcentage de Cycle spontané, Rapport l:E, Événements liés à l'inhalateur, Détection de l'État de Sommeil, Fuite buccale, Dernière exacerbation connue, Dyspnée, Niveau d'Activité, Production/couleur d'expectorations, Toux, Teneur en oxygène délivrée, Morphologie de la respiration, Température, Humidité, Qualité de l'Air, Utilisation de médicaments de secours, Température corporelle, Débit expiratoire de pointe, VEMS, et Capacité vitale ; et/ou
dans lequel les données à haute résolution comprennent l'un quelconque ou une combinaison quelconque des types de données parmi Débit Respiratoire, Pression, Événements Déclencheur/de Cycle, Pression du masque, IPAP, EPAP, Fuite, Fréquence Respiratoire, Volume Courant, Pourcentage de Déclenchement spontané, Pourcentage de Cycle spontané, Rapport l:E, Ronflement, Limitation de Débit, Fréquence du Pouls, SpO2, Temps d'inspiration, Temps d'inspiration moyen pour les 5 dernières respirations, Temps d'expiration moyen pour les 5 dernières respirations, Toux, Morphologie de la respiration, Niveau d'Activité, Débit expiratoire de pointe, VEMS, Capacité vitale, et Détection de l'État de Sommeil.

13. Dispositif de traitement de patient selon l'une quelconque des revendications 1 à 12, dans lequel le dispositif de commande peut fonctionner pour effectuer une vérification d'un événement déclencheur réel, la vérification incluant un diagnostic sur le dispositif (4000) pour déterminer si une erreur s'est produite lors du fonctionnement du dispositif, et dans lequel les données à haute résolution sont transmises sur la base de la vérification d'un événement déclencheur réel.

14. Procédé de modulation de la transmission de données de patient depuis un dispositif de traitement de patient (4000) vers un système externe (110, 412), le dispositif de traitement de patient incluant un premier capteur (4272, 4274, 4276, 4278, 4279) couplé à un patient, une mémoire (4260) et un émetteur (4280), le procédé comprenant :
la collecte d'un premier type à partir du premier capteur (4272, 4274, 4276, 4278, 4279) ;
le stockage de données à faible résolution collectées du premier capteur à un taux d'échantillonnage inférieur ;
la détermination de la survenue d'un événement déclencheur qui inclut la fourniture d'un nouveau médicament ou traitement au patient ; et
le stockage de données à haute résolution du premier capteur à un taux d'échantillonnage supérieur lors de la survenue de l'événement déclencheur ; et
la transmission soit des données à faible résolution, soit des données à haute résolution au dispositif externe via l'émetteur.
